# EUROPEAN PATENT SPECIFICATION

(11) **EP 0 587 735 B1**
(45) Date of publication and mention of the grant of the patent: **26.01.2000**
(21) Application number: 92912864.3
(22) Date of filing: 01.06.1992
(51) Int. Cl.: C07K 7/06, C07K 7/08, C07K 14/00, A61K 38/00, C12P 21/08, A61K 47/48

(54) **T CELL RECEPTOR PEPTIDES AS THERAPEUTICS FOR IMMUNE-RELATED DISEASE**
T-ZELL REZEPTORPEPTIDE ALS THERAPEUTIKA FÜR IMMUN-VERWANDTE KRANKHEITEN
PEPTIDES RECEPTEURS DE LYMPHOCYTES T UTILISES COMME AGENTS THERAPEUTIQUES CONTRE DES MALADIES DU SYSTEME IMMUNITAIRE

(30) Priority: 31.05.1991 US 708022; 16.07.1991 US 735612
(43) Date of publication of application: 23.03.1994
(73) Proprietor: CONNETICS CORPORATION, Palo Alto, CA 94303 (US)
(72) Inventor: VANDENBARK, Arthur, Allen, Portland OR 97221 (US)
(74) Representative: Moon, Donald Keith
(86) International application number: US9204492
(87) International publication number: WO9221367

(56) References cited:
- WO-A-90/11294
- WO-A-91/01133
- WO-A-92/12996
- US-A- 4 886 743
- JOURNAL OF NEUROSCIENCE RESEARCH, vol.28, no.2, February 1991, NEW YORK,USA pages 280 - 290 CHOU ET AL 'SPECIFICITY OF HUMAN T CELL CLONES REACTIVE TO IMMUNODOMINANT EPITOPES OF MYELIN BASIC PROTEIN'
- THE JOURNAL OF IMMUNOLOGY, vol.146, no.3, 1 February 1991, BALTIMORE,USA pages 854 - 859 ZHOU ET AL 'THYMIC DELETION OF VBETA11+,VBETA5+ T CELLS IN H-2E NEGATIVE,HLA-DQBETA+ SINGLE TRANSGENIC MICE'
- SCIENCE, vol.233, 22 August 1986, WASHINGTON D.C.,USA pages 879 - 883 TILLINGHAST ET AL 'STRUCTURE AND DIVERSITY OF THE HUMAN T-CELL RECEPTOR BETA-CHAIN VARIABLE REGION GENES'
- PROCEEDINGS OF THE NATIONAL ACADEMY OF SCIENCES, vol.88, October 1991, WASHINGTON D.C.,USA pages 9161 - 9165 KOTZIN ET AL 'PREFERENTIAL T-CELL RECEPTOR BETA-CHAIN VARIABLE GENE USE IN MYELIN BASIC PROTEIN-REACTIVE T-CELL CLONES FROM PATIENTS WITH MULTIPLE SCLEROSIS'
- JOURNAL OF NEUROIMMUNOLOGY, vol.37, no.1,2, March 1992, AMSTERDAM pages 123 - 129 STEVENS ET AL 'STUDIES OF VBETA8 T CELL RECEPTOR PEPTIDE TREATMENT IN EXPERIMENTAL AUTOIMMUNE ENCEPHALOMYELITIS'
- J. Neuroimmunol., Vol. 21, issued 1989, S.S. BEALL et al., "The germline repertoire of T cell receptor betachain genes in patients with chronic progressive multiple sclerosis", pages 59-66, see Abstract.
- Immunol. Rev., No. 94, issued 1986, I.R. COHEN, "Regulation of Autoimmune Disease Physiological and Therapeutic", pages 5-21, see entire article.
- Progress in Immunol. VI, issued 1986, I.R. COHEN, "Resistance to experimental autoimmunity using T lymphocyte vaccines", pages 491-499, see entire article.
- J. Neuroimmunol., Vol. 19, issued 1988, M.P. HAPP et al., "The autoreactive T-cell population in experimental allergic encephalomyelitis: T cell receptor beta-chain rearrangements", pages 191-204, see page 191.
- J. Immunol., Vol. 131, (6) issued December 1983, J. HOLOSHITZ et al., "Autoimmune encephalomyelitis (EAE) mediated or prevented by T lymphocyte lines directed against diverse antigenic determinant specific", pages 2810-2813, see Abstract.
- Science, Vol. 346, issued 03 November 1989, M.D. HOWELL et al., "Vaccination against experimental allergic encephalomyelitis with T cell receptor peptides", pages 668-670, see Abstract.
- Nature, Vol. 341, issued October 12, 1989, C.A. JANEWAY, "Immunotherapy by peptides?", pages 482-483, see entire article.
- J. Exp. Med., Vol. 167, issued May 1988, S.S. ZAMVIL et al., "Predominant expression of a T cell receptor V-Beta gene subfamily in autoimmune encephalomyelitis", pages 1586-1596, especially pages 1586 and 1595.

## Description

### BACKGROUND OF THE INVENTION

### Field of the Invention

The invention in the field of immunology and immunotherapy is directed to peptides and their pharmaceutical compositions which are capable of preventing, suppressing or treating immune-related diseases. Specifically, the invention provides a therapy that results in clinical improvement of MS patients.

### Description of the Background Art

Autoimmune diseases are characterized by an unwanted and unwarranted attack by the immune system on the tissues of the host. While the mechanism for progress of these diseases is not well understood, at least some of the details with respect to antigen presentation in this (and other) contexts is being elucidated. It is now thought that antigens, including autoantigens, are processed by antigen-presenting cells (APC), and the resulting fragments are then associated with one of the cell surface proteins encoded by the major histocompatibility complex (MHC). As a result, recognition of a peptide antigen is said to be MHC "restricted." When the MHC/antigen fragment complex binds to a complementary T cell receptor (TCR) on the surface of a T lymphocyte, it leads to activation and proliferation of the clone or subpopulation of T cells that bear that particular TCR. Once activated, T cells have the capacity to regulate other cells of the immune system which display the processed antigen and to destroy cells or tissues which carry epitopes of the recognized antigen.

A review of the role of TCRs in autoimmune diseases by Acha-Orbea *et al. (Ann. Rev. Immunol. 7*:371-405 (1989)) discussed the tremendous variation in TCRs available in the immune system of an individual and the generation of this diversity by germ line gene organization and rearrangement of the DNA encoding TCR α and β chains. The α chains are encoded by various combinations of variable (V), junction (J) and constant (C) region gene segments. TCR β chains are additionally encoded by a diversity (D) region gene segment, and, thus comprise a rearranged VDJC sequence. Due to allelic exclusion, a clone of T cells expresses only one type of TCR α-β heterodimer.

A growing number of human diseases have been classified as autoimmune in nature (see, Theofilopoulos, A., In: D.P. Stites *et al.* eds., *Basic and Clinical Immunology*, Lange Medical Publications, Los Altos, CA, 1988) of which several examples are rheumatoid arthritis (RA), myasthenia gravis (MG), multiple sclerosis (MS), systemic lupus erythematosus (SLE), autoimmune thyroiditis (Hashimoto's thyroiditis), Graves' disease, inflammatory bowel disease, autoimmune uveoretinitis, polymyositis and certain types of diabetes. Animal models have been developed for a number of these human autoimmune diseases. Among the best studied model is experimental allergic encephalomyelitis (EAE, also called experimental autoimmune encephalomyelitis), a model for MS.

Because it is now known that these and other autoimmune diseases involve the action of T helper cells stimulated by the binding of their TCR to an MHC/autoantigen (or non-autoantigen) complex, prevention and/or treatment strategies have been proposed which are based on the disruption of interactions between the MHC/antigen complex and the TCR. Wraith, D.C. *et al., Cell 57*:709-715 (1989)), proposed approaches based on this principle, including vaccination with whole T cells (as initially described by I.R. Cohen's laboratory, discussed below), passive blockade using antibodies which bind to the TCR, passive blockade using antibodies that bind to the MHC portion of the complex, administration of antibodies reactive with the T helper cell marker, CD4, and the use of peptides which mimic the antigen of interest and compete for binding to the MHC or the TCR molecule.

Myelin basic protein, MBP, is the major autoantigen involved in EAE and is the leading candidate as an encephalitogen involved in MS.

Heber-Katz's group (Heber-Katz, E. *et al., Ann. N.Y. Acad. Sci. 540*:576-577 (1988); Owhashi *et al., J. Exp. Med. 168*:2153-2164 (Dec. 1988)) has analyzed the fine specificity of recognition of MBP epitopes by rat T cells. When T cells from rats immunized with MEP were hybridized to a mouse T lymphoma line and cloned, the pattern of fine specificity and Southern blot analysis of the TCR Vβ gene rearrangement indicated a polyclonal response, even though 75% of the clones reacted to the 68-88 encephalitogenic determinant. A monoclonal antibody (mAb), designated 10.18, directed at one encephalitogenic T cell hybridoma proved to be an anti-idiotype or anti-clonotype which reacted only with T cell clones specific for the MBP 68-88 epitope. The mAb could block or reverse EAE when injected with, or 5 days after, the encephalitogenic MBP peptide. Soluble mAb 10.18 blocked the specific T cell clones, and immobilized mAb 10.18 stimulated their proliferation. Following induction of EAE with MBP, the proportion of mAb 10.18-binding cells increased from initially very low frequencies. The authors concluded that the 10.18⁺ T cells probably represent the dominant pathogenic T cell repertoire of EAE in Lewis rats. However, it was not known whether mAb 10.18 recognized a V region or an idiotypic determinant.

T cells expressing the TCR αβ heterodimer can induce idiotypic and V gene family-specific antibodies that can regulate T cell function (Owhashi *et al., supra*; Gascoigne *et al., Proc. Natl. Acad. Sci,* USA *84:*2936 (1987); Kappler *et al., Nature 332*:35 (1988); Kappler *et al., Cell* 49:263 (1987); MacDonald *et al., Nature 332*:40 (1988)). For example, antibodies that recognize the TCR Vβ8 sequence have been effective in the prevention and treatment of autoimmunity in mice and rats (Owhashi *et al., supra*; Acha-Orbea *et al., Cell 54*:263-273 (1988); Urban *et al., Cell 54*:577-592 (1988)). Obtaining such antibodies selective for V region gene products has been dependent upon the availability of T cell clones that express TCR encoded by the relevant V gene family, and requires a laborious screening procedure using whole cells to establish specificity.

While antibody therapies in which antibodies are directed to MHC molecules and CD4 molecules have been generally successful in several animal models of autoimmunity, these approaches may be too nonspecific and potentially overly suppressive, since 70% of T cells bear the CD4 marker, and since all T cell-mediated responses and most antibody responses require MHC-associated antigen presentation.

Multiple sclerosis (MS) is an immune-mediated disease characterized by central nervous system mononuclear cell infiltration and demyelination. Although the pathogenesis of MS is unknown, both genetic and environmental factors have been implicated in the disease process. Major elements of the genetic predisposition include an association of disease with particular class II major histocompatibility complex (MHC) halotypes, in particular HLA-DR21 and -DQw1 (Terasaid *et al., Science 1933*:1245-1247 (976); Ho *et al., Immunogenetics 15*:509-517 (1982); Spielman *et al., Epidemiol. Rev. 4*:45-65 (1982); Francis *et al., Lancet 1*:211 (1986); Elian *et al., Disease Markers 5*:89-99 (1987)), as well as with certain polymorphisms within the T cell receptor (TCR) α-chain and β-chain gene complexes (Beall *et al., J. Cell. Biochem. 11D*:223 (1987); Hauser *et al., J. Neurot 89*:275-277 (1989); Seboun *et al., Cell 57:*1095-1100 (1989)). These studies suggest that the disease involves CD4⁺T-cells bearing αβ TCR. In support of this idea, CD4⁺T cells represent a major component of mononuclear cells in the brains of active patients α-chain T cell receptors are present within central nervous system tissue of MS patients but not controls (Terasaid *et al., Science 193*:1245-1247 (976).

T lymphocytes that recognize myelin basic protein (BP) have been shown to have potent demyelinating and encephalitogenic activity in animals (Ben-Nun *et al., Eur. J. Immunol. 11*:195-199 (1981); McFarlin *et al., New Eng. J. Med. 307*:1183-1188 (1982); Mokhtarian et al., Nature *309*:356-358 (1984); Vandenbark *et al., J. Immunol. 135*:223-228 (1985); Zamvil *et al., Nature 317*:355-358 (1985); Bourdette *et al., Cell Immunol. 112*:351-363 (1988). Accumulating evidence also suggests that BP-specific T cells may contribute to the pathogenesis of MS. Thus, cells selected from MS patients on the basis of *in vivo* activation have specificity for BP. The frequencies of BP-reactive T cells are also increased in the blood and cerebrospinal fluid (CSF) of MS patients compared to normal individuals or patients with other neurological diseases. Furthermore, recent studies have demonstrated a marked selective enrichment of BP-reactive T cells in the CSF relative to the blood of individual MS patients. In animals, a limited set of TCR α-chain variable (Vα) and β-chain variable (Vβ) genes are utilized by T cells specific for BP (Acha-Orbea *et al., Cell 54*:263-273 (1988); Urban *et al., Cell 54*:577-592 (1988); Burns *et al., J. Exp. Med. 169*:27-39 (1989); Heber-Katz *et al., Immunol. Today 10*:164-169 (1989)). Monoclonal antibodies directed to these regions or synthetic peptides with sequences common to these TCR variable regions can both protect and treat animals with clinical signs of experimental autoimmune encephalomyelitis (EAE) (Acha-Orbea *et al., Cell 54*:263-273 (1988); Urban *et al., Cell 54*:577-592 (1988); Vandenbark *et al., Nature 341*:541-544 (1989); Howell *et al., Science 246*:668-670 (1989)). In order for a similar approach to be applied to MS patients, it is critical to know if potentially pathogenic T cells also preferentially utilize a limited set of V region genes.

I.R. Cohen's laboratory has developed an approach to the immunospecific treatment of autoimmunity which utilizes whole live or attenuated T lymphocytes as vaccines to treat or prevent EAE, experimental autoimmune thyroiditis (EAT), and experimental arthritis. This approach is reviewed in Cohen, I.R., *Immunol. Rev. 94*:5-21 (1986), which discusses several animal models of autoimmune disease wherein vaccination with disease-specific T lymphocytes has been used to generate prophylactic or therapeutic effects. The fine specificity of vaccination was dictated by the fine specificity of the T cell recognition, possibly implicating the TCR. For example, two different anti-MBP T cell lines, each reactive to a different epitope of MBP, were found to vaccinate against EAE specifically induced by the particular epitope, indicating some form of anti-idiotypic immunity. However, when attempts were made to isolate clones of MBP-specific or thyroglobulin-specific T cells (in a thyroiditis model) from the non-clonal cell lines, only clones producing disease, but not resistance, were obtained. This led to the finding that appropriate aggregation or rigidification of cell membranes, by either hydrostatic pressure or chemical cross-linking, yielded cells which could induce protection more consistently. Similarly, low doses (sub-encephalitogenic) of MBP-specific cells could also induce resistance to lethal EAE. The protective state was termed "counter-autoimmunity." This state involves T cell clones which can specifically proliferate in response to the vaccinating T cells, can suppress effector clones *in vitro* (non-specifically, presumably through release of a suppressive lymphokine), and can adoptively transfer counter-autoimmunity *in vivo.* Such counter-autoimmunity is accompanied by suppressed delayed hypersensitivity (DH) responses to the specific epitope and prevention or remission of clinical disease.

A major difficulty with the foregoing approaches is that they require the use of complex biological preparations which do not comprise well-defined therapeutic agents. Such preparations suffer from complex production and maintenance requirements (e.g., the need for sterility and large quantities of medium for producing large number of "vaccine" T cells), and lack reproducibility from batch to batch. The T cell "vaccine" preparations, to be useful in humans, must be both autologous and individually specific, that is, uniquely tailored for each patient. Furthermore, the presence of additional antigens on the surface of such T cells may result in a broader, possibly detrimental, immune response not limited to the desired T cell clones (Offner *et al., J. Neuroimmunol. 21*:13-22 (1989).

There is a great need, therefore, for agents and pharmaceutical compositions which have the properties of specificity for the targeted autoimmune response, predictability in their selection, convenience and reproducibility of preparation, and sufficient definition to permit precise control of dosage.

Currently, no effective treatment for MS is known. (*Harrison's Principles of Internal Medicine*, 12th ed. Wilson *et al.*, McGraw Hill, Inc. 1991). Therapeutic efforts are directed toward amelioration of the acute episode, prevention of relapses or progression of the disease, and relief of symptoms. The clinical manifestations of MS depend upon which nerve group or region of the brainstem, cerebellar or spinal cord is involved. Spinal cord involvement is the predominating feature in most advanced cases of MS.

In acute episodes of disease, glucocorticoid treatment has been suggested as having the potential to lessen the severity of symptoms and speed recovery, however, even its proponents point out that ultimate recovery is not improved by this drug nor is the extent of permanent disability altered. ACTH is the preferred glucocorticoid of clinicians since the only controlled trials which demonstrated any efficacy of glucocorticoid therapy in episodes of MS and optic neuritis were performed with this drug. However, use of long term steroids is not advised.

Immunosuppressive agents such as azathioprine and cyclophosphamide have been claimed to reduce the number of relapses in several series, but there is no consensus about the efficacy of these drugs either.

The current recommendations for the treatment of MS revolve around attempting to avoid exacerbation of the symptoms. Patients are advised to avoid excess fatigue and extremes of temperature and eat a balanced diet. (The above discussion is primarily from Chapter 356 of *Harrison's Principles of Internal Medicine*, 12th ed 1991.)

WO90/11294 proposes the use of T cells, T cell receptors or fragments thereof as vaccines against T cell mediated pathologies.

### SUMMARY OF THE INVENTION

This invention was made in response to a clear need for therapeutic agents and compositions capable of preventing, suppressing or treating immune-related diseases in a clone-specific manner, without causing generalized suppression of immunity, as is the case with most current immunotherapeutic and immunopharmacologic approaches. The invention was developed from the knowledge that lines or clones of T cells specific for autoantigens, which actually mediated autoimmune disease, could be converted into therapeutics by complex attenuation protocols, and injected directly into animals to prevent or treat the disease.

Accordingly the invention provides a peptide consisting of an amino acid sequence corresponding to a portion of a T cell receptor, wherein the T cell receptor is a marker T cell receptor characteristic of a T cell mediated disease, the peptide being capable of stimulating regulatory T cells and inducing protection from the disease, wherein the peptide is selected from the group consisting of human Vβ5.2 (26-43), human Vβ5.2 (39-59) and human Vβ5.2 (59-78), human Vβ6.1 (1-22), human Vβ6.1 (39-59) and human Vβ6.1 (70-88) or a peptide substantially similar to a said peptide or a fragment thereof with the proviso that the "peptide substantially similar to a said peptide" and the "fragment thereof" are also capable of stimulating regulatory T cells and inducing protection from the disease.

In another embodiment the invention provides a peptide consisting of an amino acid sequence corresponding to a portion of a T cell receptor, wherein the T cell receptor is a marker T cell receptor characteristic of a T cell mediated disease, the peptide being capable of stimulating regulatory T cells and inducing protection from the disease, wherein the amino acid sequence is selected from the group consisting of the consensus sequence ALGQGPXFX, the consensus sequence LRLIHYSYDVN and the consensus sequence DPGXGLRLIXYS or a peptide substantially similar to a said peptide or a fragment thereof with the proviso that the "peptide substantially similar to a said peptide" and the "fragment thereof" are also capable of stimulating regulatory T cells and inducing protection from the disease.

In a further embodiment the invention provides a peptide consisting of an amino acid sequence corresponding to a portion of a T cell receptor, wherein the T cell receptor is a marker T cell receptor characteristic of a T cell mediated disease, the peptide being capable of stimulating regulatory T cells and inducing protection from the disease, wherein the amino acid sequence is selected from the group consisting of the sequence ALGQGPQFIFQYYEEEERQRG, the sequence LGQGPEFLIYFQGTGAADDSG, the sequence DPGHGLRLIHYSYGVKDTDKG, the sequence DPGQGLRLIYYSQIVNDF QKG and the sequence DPGLGLFRQIYYSMNVEVTDKG or a peptide substantially similar to a said peptide or a fragment thereof with the proviso that the "peptide substantially similar to a said peptide" and the "fragment thereof" are also capable of stimulating regulatory T-cells and inducing protection from the disease.

The invention also encompasses the peptide conjugated to a carrier, such as an additional heterologous amino acid sequence, in order to enhance the peptide's immunogenicity.

The invention is also directed to a pharmaceutical composition comprising one or more of the peptides in admixture with a pharmaceutically acceptable excipient.

Thus, the invention provides chemically defined peptides and therapeutics which can be specifically applied to designated immune-related diseases to disrupt the specific immunological responses responsible for induction or promotion of the disease process.

The diseases for which the invention is particularly useful include autoimmune diseases, such as rheumatoid arthritis, adjuvant arthritis, myasthenia gravis, encephalomyelitis, multiple sclerosis, thyroiditis, diabetes, inflammatory bowel disease and systemic lupus erythematosus. The invention is also directed to malignant disease, such as T cell leukemias and lymphomas wherein the TCR serves as a tumor marker.

### BRIEF DESCRIPTION OF THE DRAWINGS

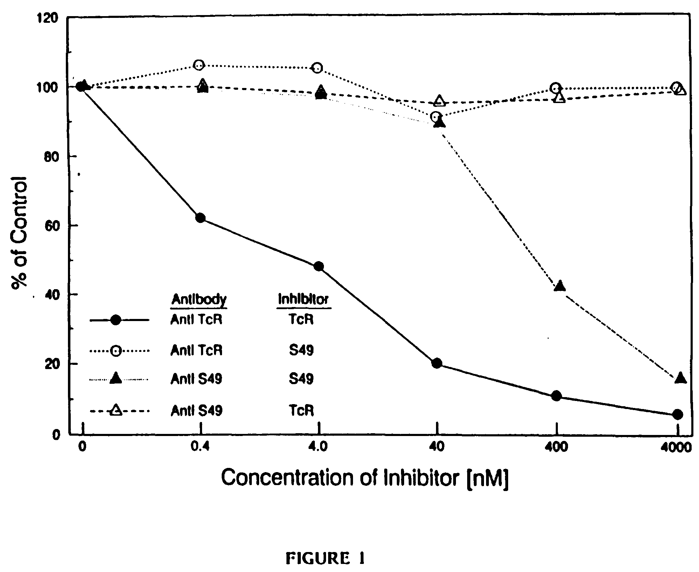

**Figure 1.** Peptide-specific inhibition of antibody reactivity. Antisera from 4 rats immunized with either the TCR Vβ8(39-59) peptide alone or a combination of the TCR peptide and the GP-S49S MBP-derived autoantigen were pooled and diluted 40-360 fold. The antisera were tested for reactivity in direct ELISA with 25 ng peptide bound to microplate wells. This amount of peptide is equivalent to 10 pM TCR Vβ8(39-59) (MW = 2390 daltons) or 15 pM GP-S49S (MW = 1630 daltons). Varying concentrations of inhibitor peptides were added in a range of 0.005 - 50 µg/well. The Absorbance measurements were determined in triplicate wells, and the reactivity calculated as the % of uninhibited control wells.

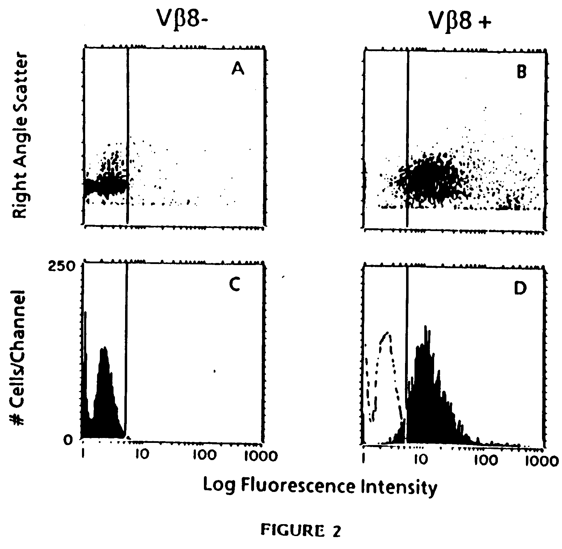

**Figure 2.** Antibodies to the TCR Vβ8(39-59) peptide stain Vβ8⁺ encephalitogenic T cells. Normal thymocytes (A and C) or GP-S49S-specific T line cells (B and D) were incubated with rabbit antibodies to TCR Vβ8(39-59), followed by a mouse anti-rabbit IgG facilitating antibody and fluorescein-labeled goat anti-mouse IgG antibody. Flow cytometric analysis of staining was performed using a Coulter Epics C Cytofluorograph. A and C represent dot-plots of 10,000 cells showing cell size versus fluorescence intensity; B and D represent the corresponding histograms. The fluorescence intensity of T line cells stained with anti-TCR Vβ8(39-59) antibody (>90% stained) is increased compared to the 5% of normal thymocytes which stained with this antibody. Both thymocytes and T line cells incubated with normal rabbit IgG as a control for anti-TCR Vβ8(39-59) IgG showed background levels of staining (dotted line in box D).

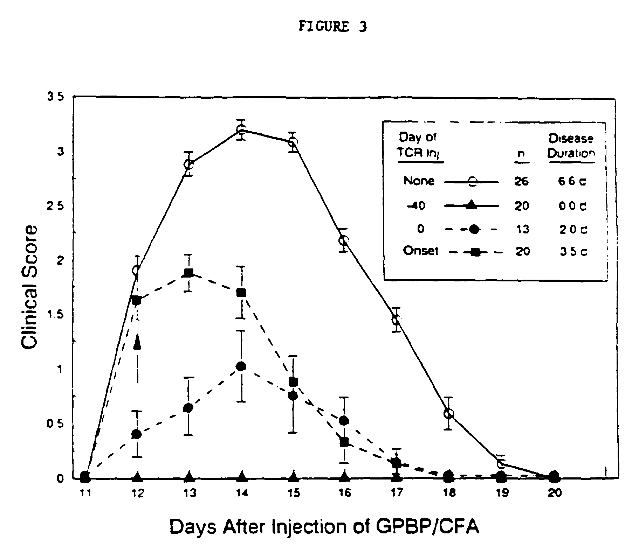

**Figure 3.** Prevention, suppression and treatment of EAE with 50 µg TCR Vβ8-39-59 peptide/CFA. Rats were injected s.q. with the TCR peptide 40 days prior to, at the same time, or at disease onset 12 days after the induction of EAE with 50 µg GP-MBP/CFA.
**Figure 4.** Suppression of EAE with 50 µg TCR Vβ8-39-59 peptide given i.d. in the ear at the same time, or on days 7 or 11 after induction of EAE with 50 µg GP-MBP/CFA.

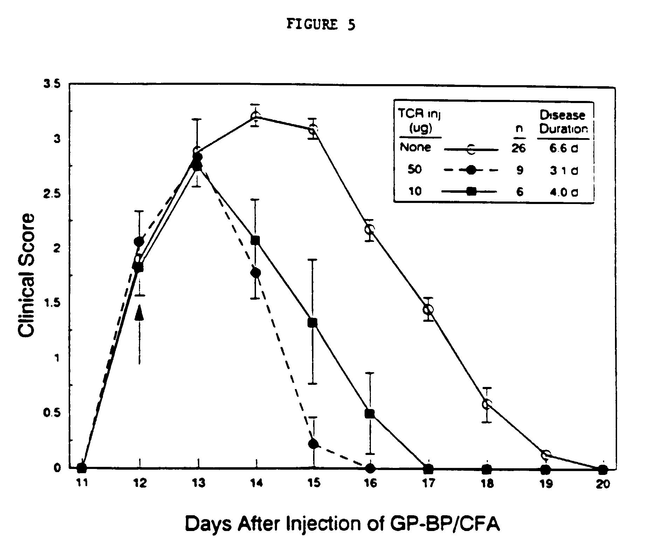

**Figure 5.** Treatment of EAE with 10 or 50 µg TCR Vβ8-39-59 peptide given i.d. in the ear at onset of clinical signs (day 12) after induction of EAE with 50 µg GP-MBP/CFA.

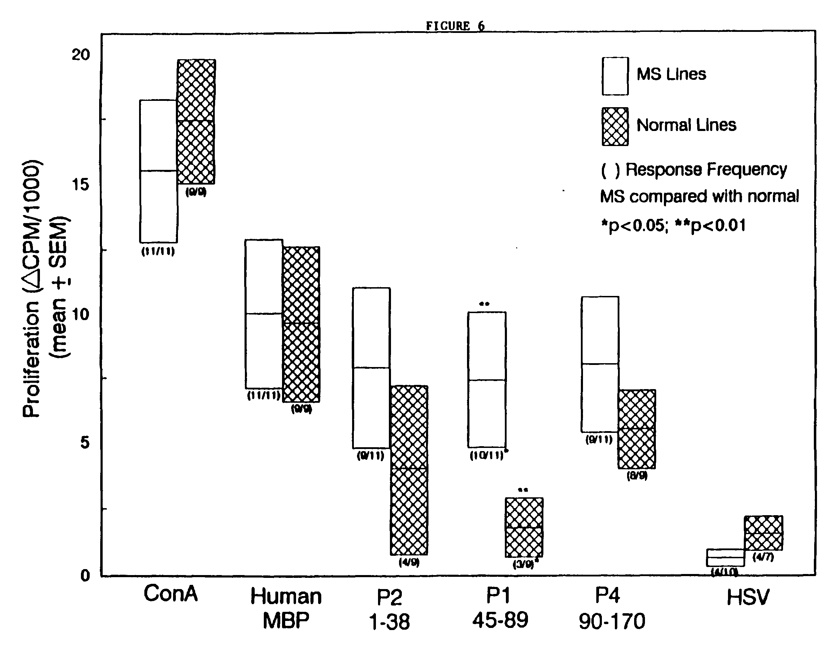

**Figure 6.** Peptide specificity of human MPB-specific T cell lines from MS patients and normals.

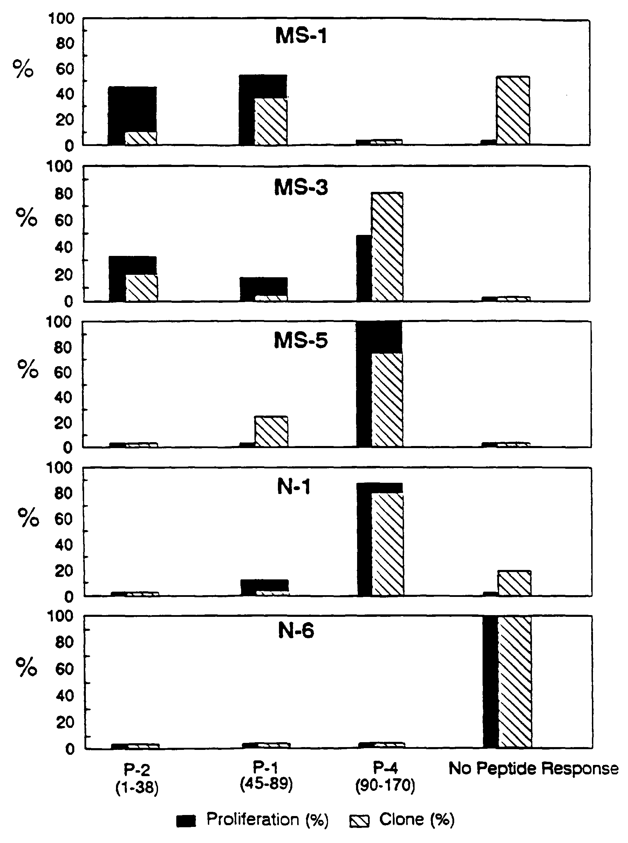

**Figure 7.** Percentage of total proliferation response of T cell lines directed at each peptide compared to percentage of total clones responding to each peptide.

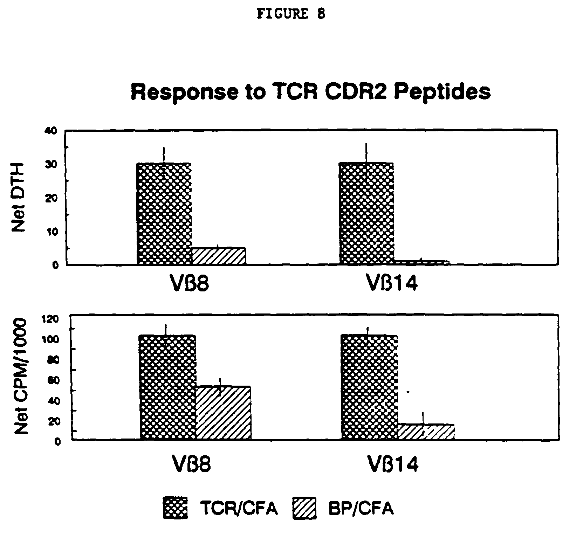

**Figure 8.** Cellular responses to TCR Vβ8- and Vβ14 peptides from EAB-recovered and TCR peptide-immunized rats. DTH is given in mm/100 and proliferation in CPM/1000, both background subtracted.

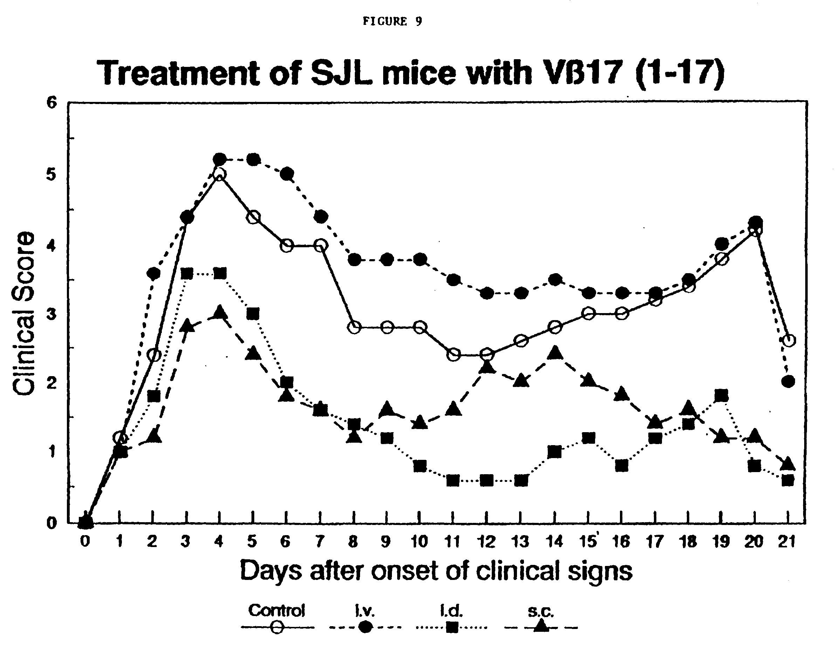

**Figure 9.** Treatment of relapsing EAE with TCR Vβ17 peptide.

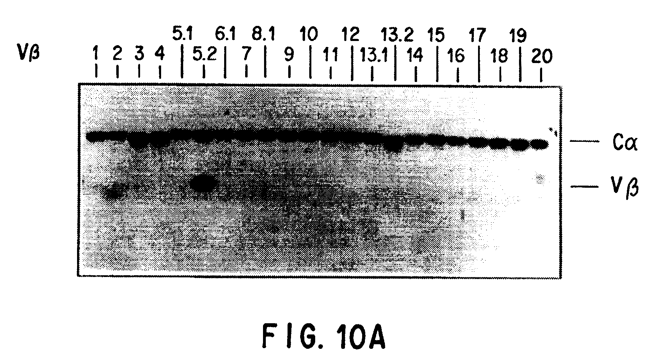

**Figure 10A.** Analysis of TCR Vβ expression in clone #41 from patient MR. Autoradiogram shows TCR products amplified by PCR. Total RNA was prepared from the cloned T cells and used for the synthesis of first strand cDNA as described (Choi *et al., Proc. Natl. Acad. Sci.* USA *86*:8941-8945 (1989)). Each PCR reaction contained specific oligonucleotide primers to expand the particular Vβ gene segment indicated (170-220 bp) as well as a Cα gene segment (^{∼} 600 bp). The sequences of the specific primers used and details of the PCR have been described (Choi *et al., Proc. Natl. Acad. Sci.* USA *86*:8941-8945 (1989)). Amplified products were separated on 2% agarose gels, dried, and exposed to x-ray film. Incorporation of ³²P end-labeled 3' primers was used to identify amplified products.
   Vβ5.2 is the only positive Vβ band in this autoradiogram. The primer used for amplification of Vβ5.2 cDNA is specific for a sequence common to Vβ5.2 and 5.3 (Choi *et al., Proc. Natl. Acad. Sci.* USA *86*:8941-8945 (1989)). Control PCR reactions without added cDNA template but with oligomers specific for Vβ5.2/5.3 showed no Vβ bands.

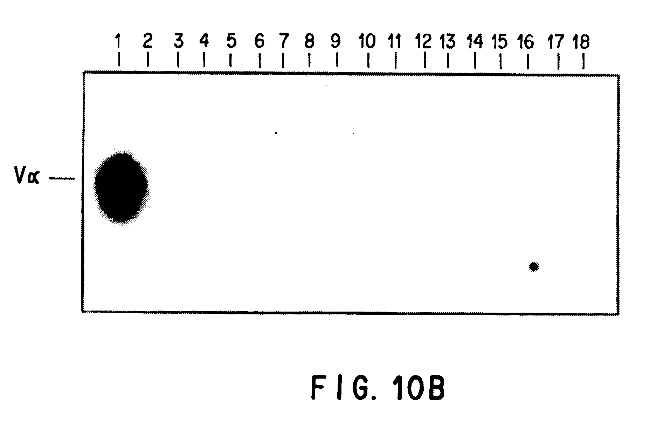

**Figure 10B****.** Analysis of TCR Vα expression of clone #41 from patient MR. Autoradiogram shows detection of PCR-amplified Vα products. Methods are similar to those described for Figure 10A except that Vα cDNA was amplified using Vα-specific primers as described (Oksenberg *et al., Nature 345*:344-346 (1990)). In addition, after gel separation and transfer to nylon membranes, Southern Blots were hybridized by the use of a ³²P-kinase probe (5'-AATATCCAGAACCCTGACCCT-3') corresponding to an internal Cα region (Oksenberg *et al., Nature 345*:344-346 (1990)) and autoradiographed. The size of amplified Vα products ranged from about 320-420 base pairs (Oksenberg *et al., Nature 345*:344-346 (1990)). Vα1 is the only positive band in this Southern blot. For some experiments, slot blots rather than Southern blots were hybridized with the labeled Cα probe. Both detection techniques gave identical results.

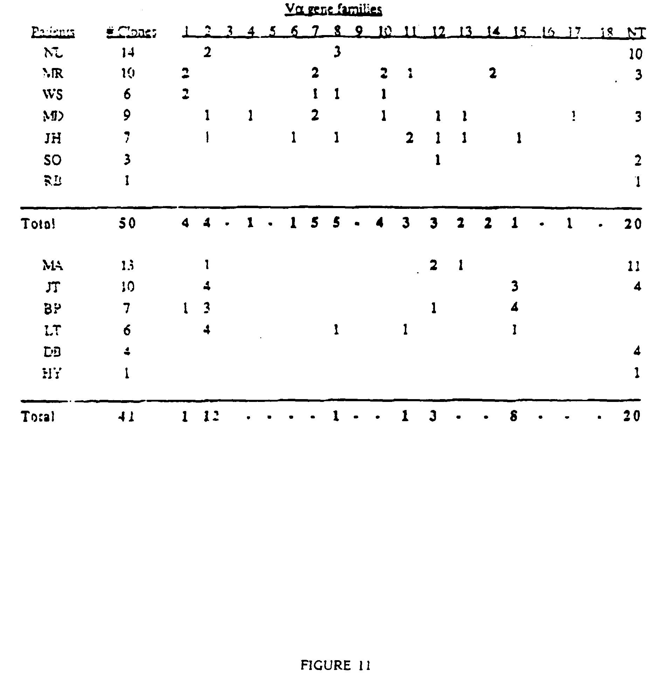

**Figure 11.** Summary of usage of Vα genes in BP-reactive T cell clones from MS patients and normal individuals. Methods are those as described in Figure 1B and Tables 1 and 2. Only the predominant Vα band for each clone is included in this summary analysis. Since some clones had two bands of nearly equal intensity, the total a number of Vαs may be greater than the number of clones analyzed. NT indicates the number of clones that were not tested for Vα expression.

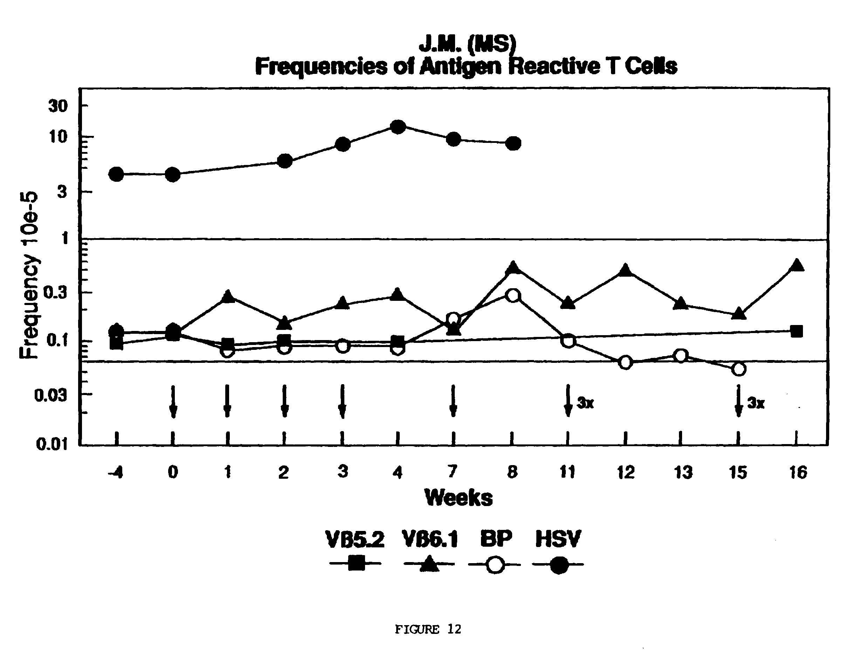

**Figure 12****.** Frequencies of antigen reactive T cells in MS patient J.M.

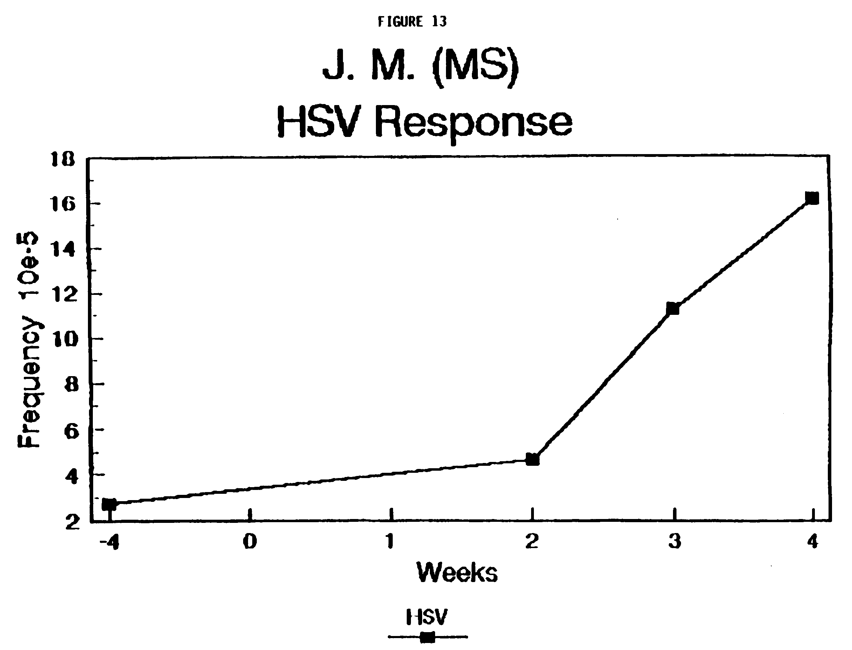

**Figure 13****.** HSV response in J.M.

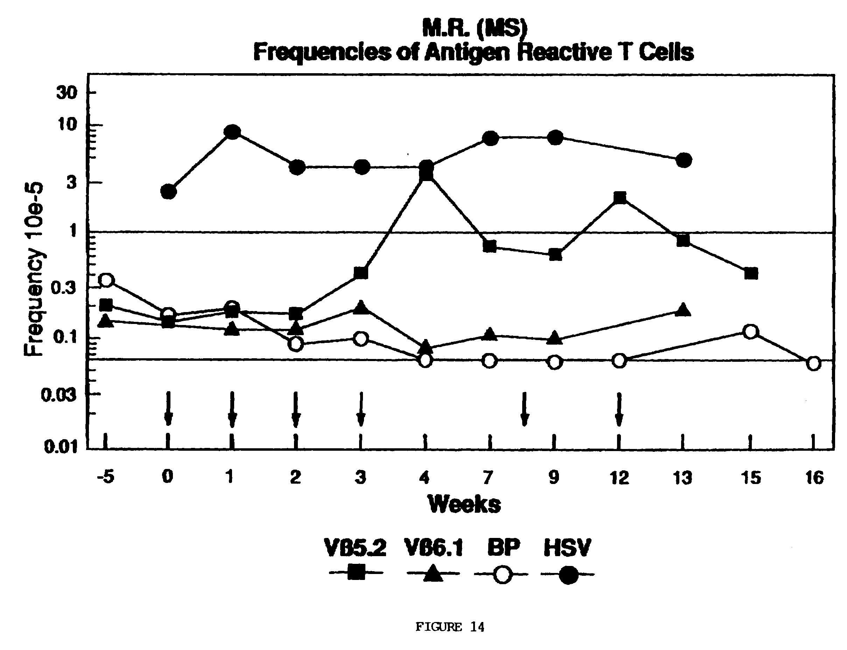

**Figure 14****.** Frequencies of antigen reactive T cells in MS patient M.R.

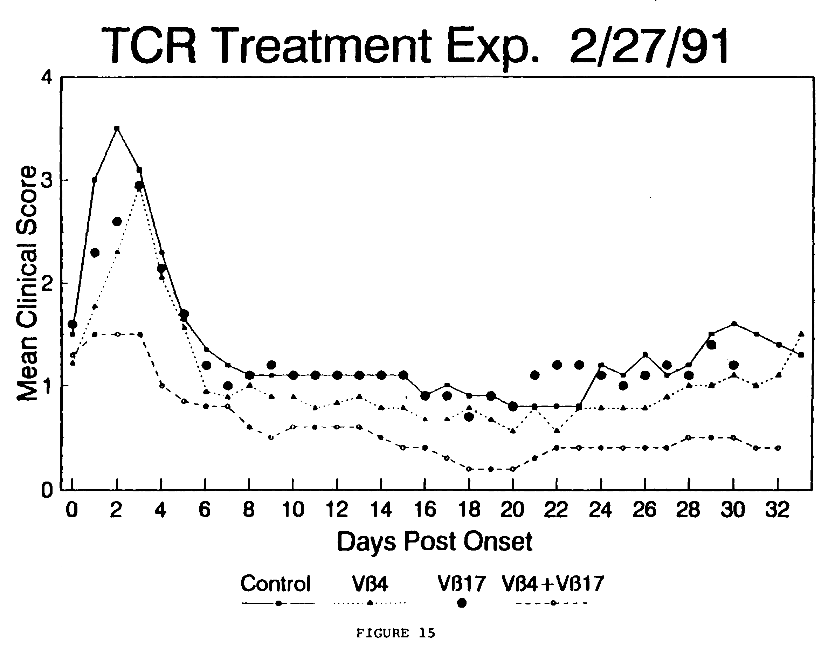

**Figure 15****.** TCR treatment in SJL mouse. PLP (proteolipoprotein) was used as the antigen to induce EAE.

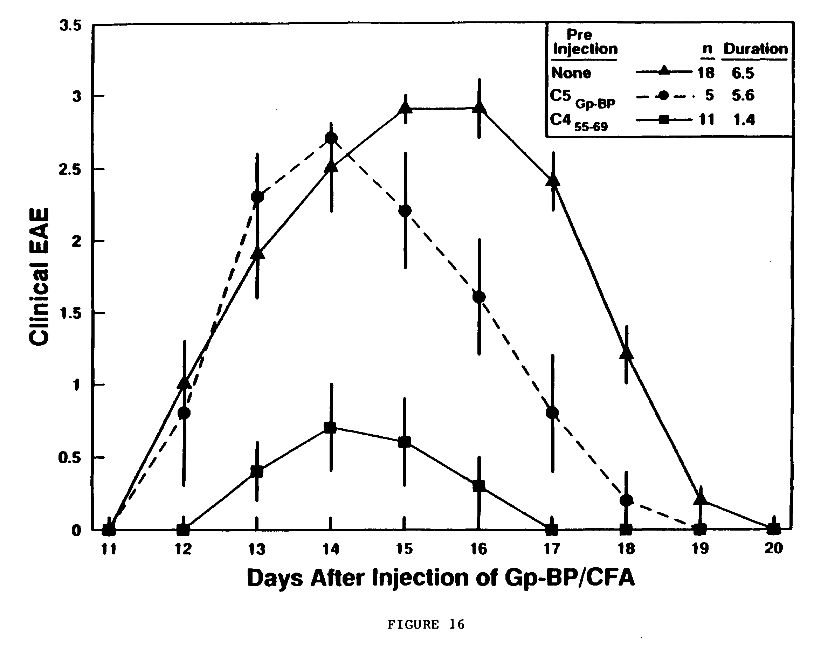

**Figure 16****.** Pre-immunization with Gp-BP-55-69 inhibits the induction of EAE. Rats were injected with either 100 µg Gp-BP-55-69/CFA or CFA alone for 4 weeks prior to challenge with 10 µg Gp-BP/CFA. Clinical EAE was assessed daily in both groups according to the scale described in Methods (Example XIV).

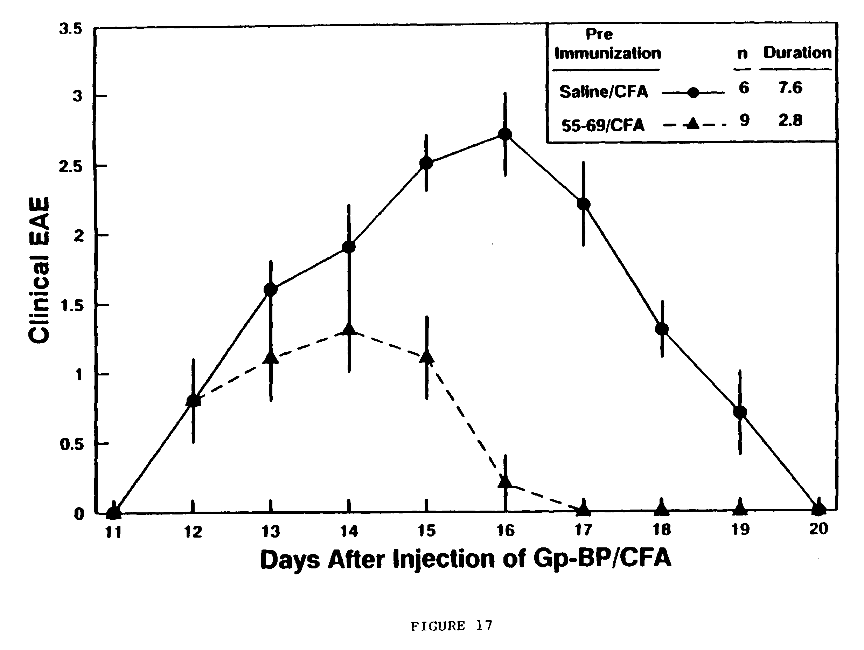

**Figure 17****.** Passive transfer of clone C4₅₅₋₆₉inhibits the induction of EAE. Rats were injected with 10 million activated C4₅₅₋₆₉ or C5_{Gp-BP} cloned T cells for 14 days prior to challenge with Gp-BP/CFA.

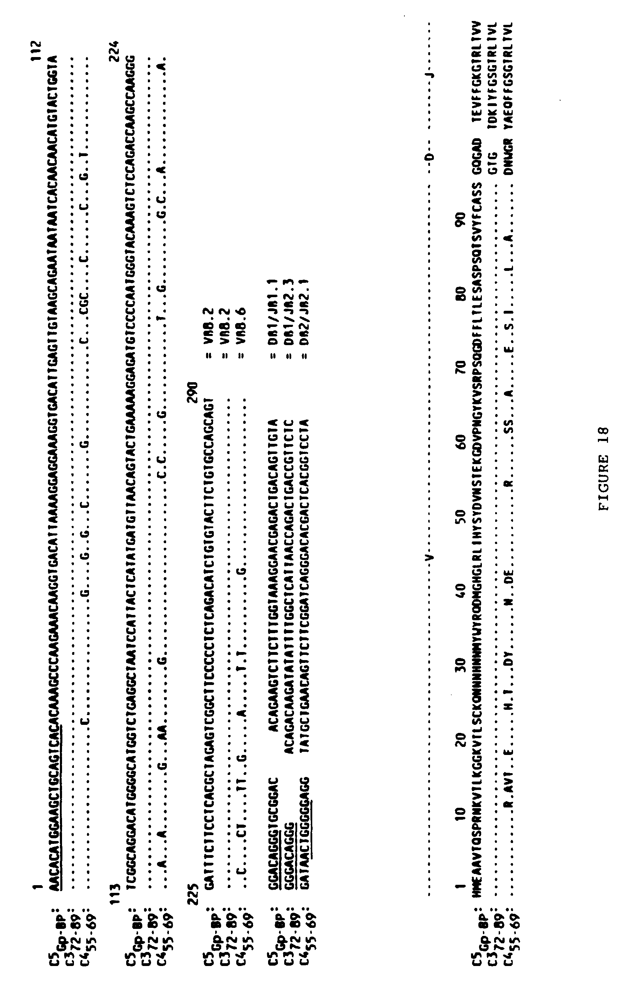

**Figure 18****.** Nucleotide (upper section) of TCR-Vβ chains utilized by anti-MBP clones. Bases 1-22, shown in italics, are derived from oligonucleotide used in PCR amplification (see Methods, Example XIV). Underlined bases in the D region indicate germline sequences, and the remaining bases indicates N region additions. Lower section indicates predicted amino acid sequence of TCR-Vβ chains used by anti-MBP clones. Bases and amino acid residues that differ from the Vβ8.2 sequence are indicated below the sequence for clone C5_{Gp-BP}.

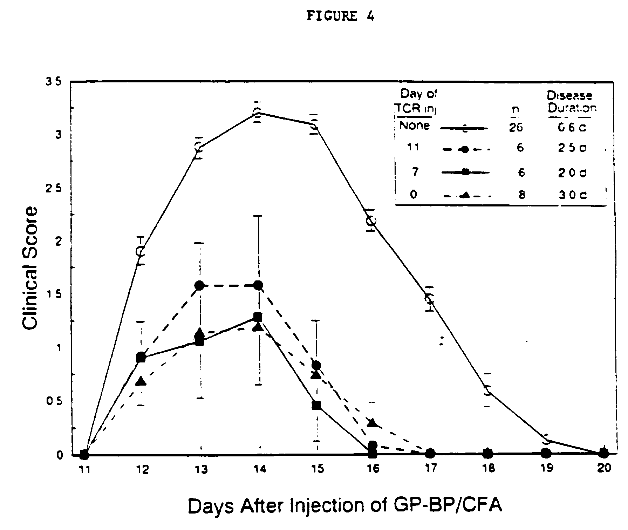

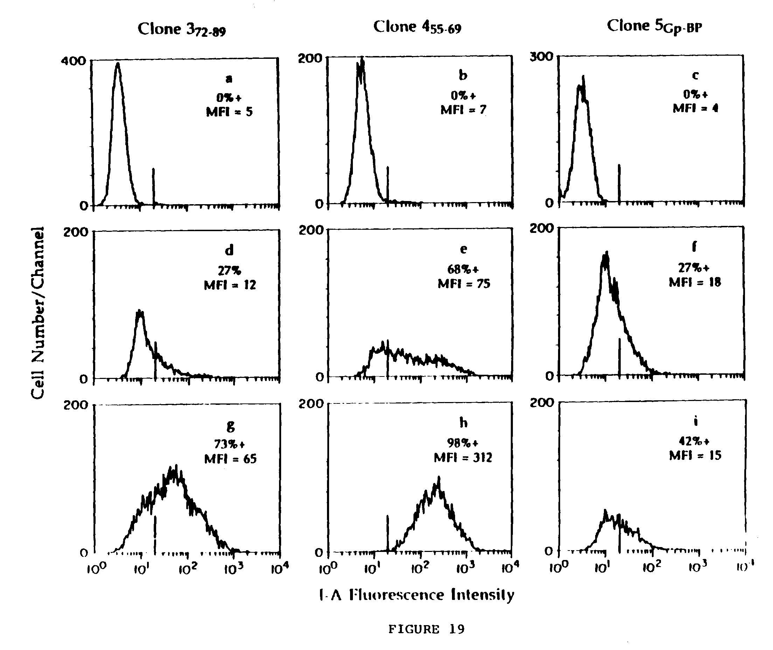

**Figure 19****.** Expression of I-A by activated and resting T cell clones from EAE-recovered rats. Cells were stained with OX-6 and counterstained with FITC-conjugated goat anti-rat IgG either 3 days after activation with Gp-BP (4g-i) or after an additional 7 days growth in IL-2 rich supernatant (4d-f). Figures 4a-c indicate the background level of staining with isotype control antibody.

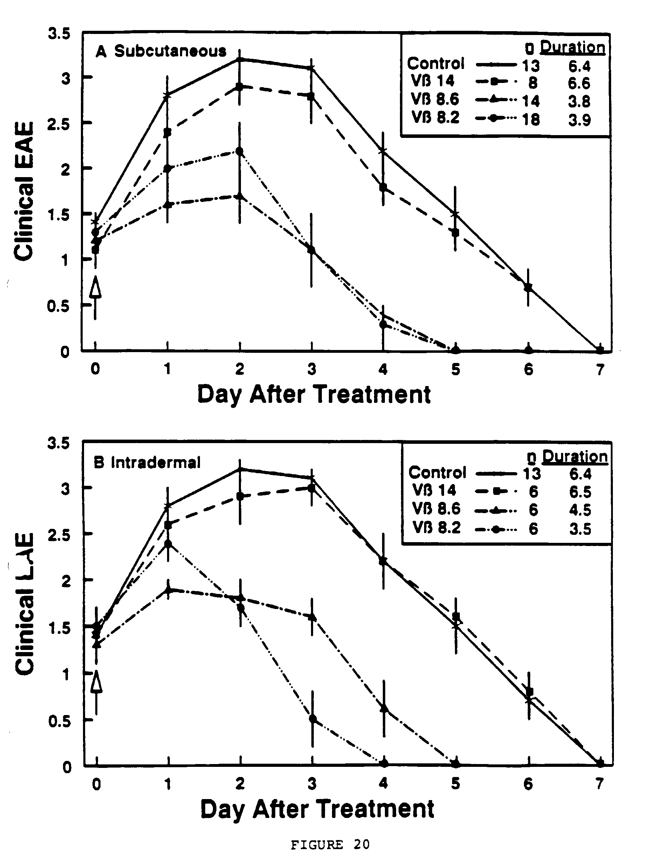

**Figure 20****.** Treatment of EAE with TCR Vβ8.6₃₉₋₅₉ and TCR Vβ8.2₃₉₋₅₀ peptides. Rats were injected with the indicated doses of TCR peptides either subcutaneously (5a) or intradermally (5b) on the first day of onset of clinical signs, usually day 12 after injection of Gp-BP/CFA. Control groups received TCR Vβ14₃₉₋₅₉ peptide or no peptide.

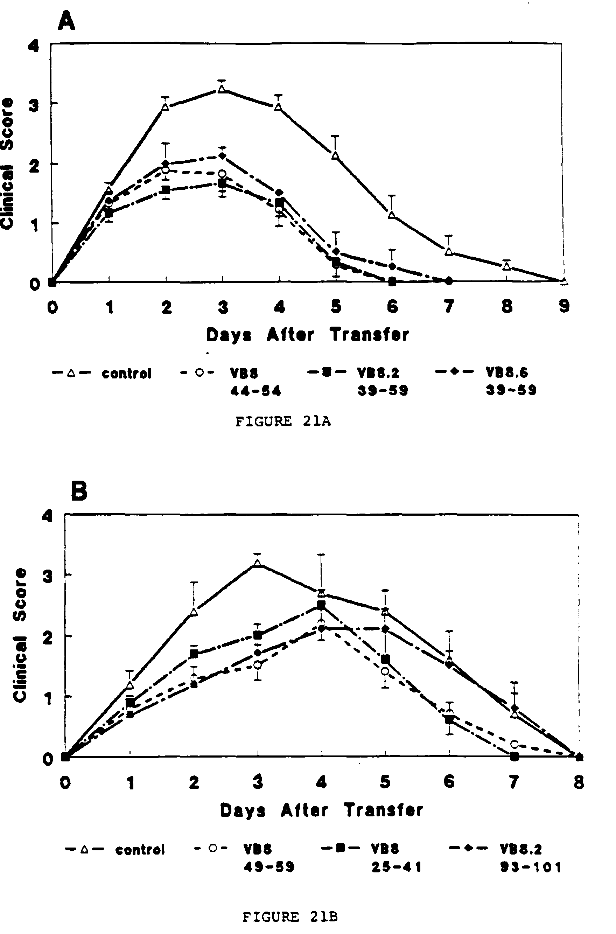

**Figure 21****.** Treatment of actively induced EAE with TCR Vβ8 peptides.

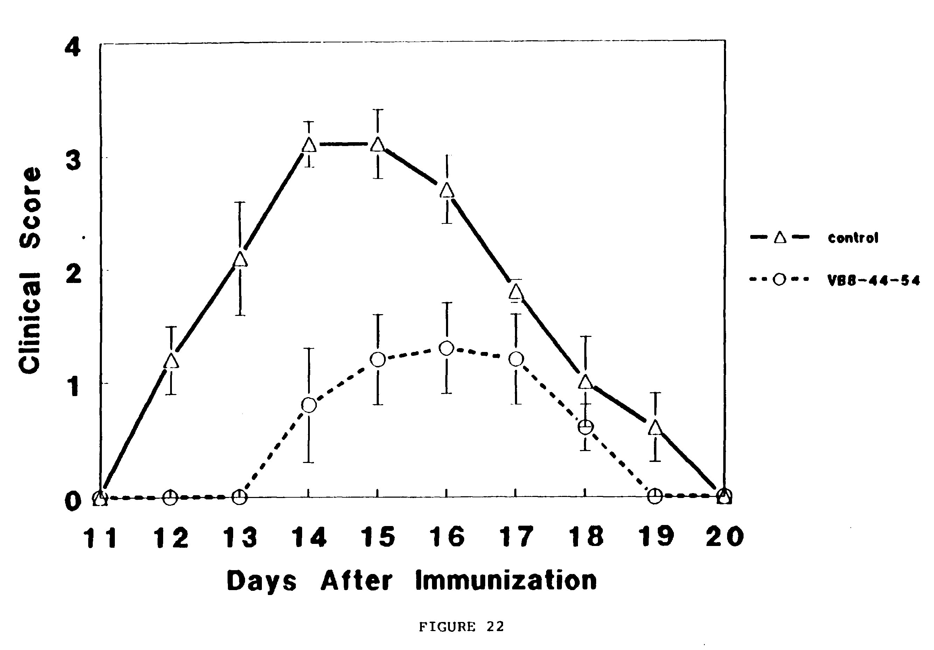

**Figure 22****.** Treatment of passively induced EAE with TCR Vβ8 peptides.

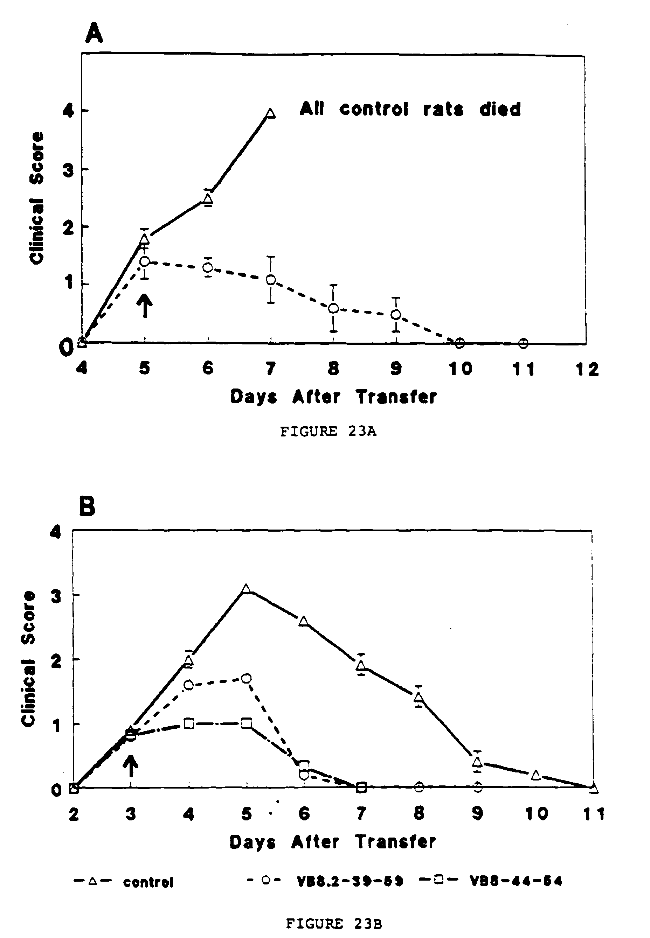

**Figure 23****.** T line cells specific for TCR Vβ-44-54 transfer protection against EAE.

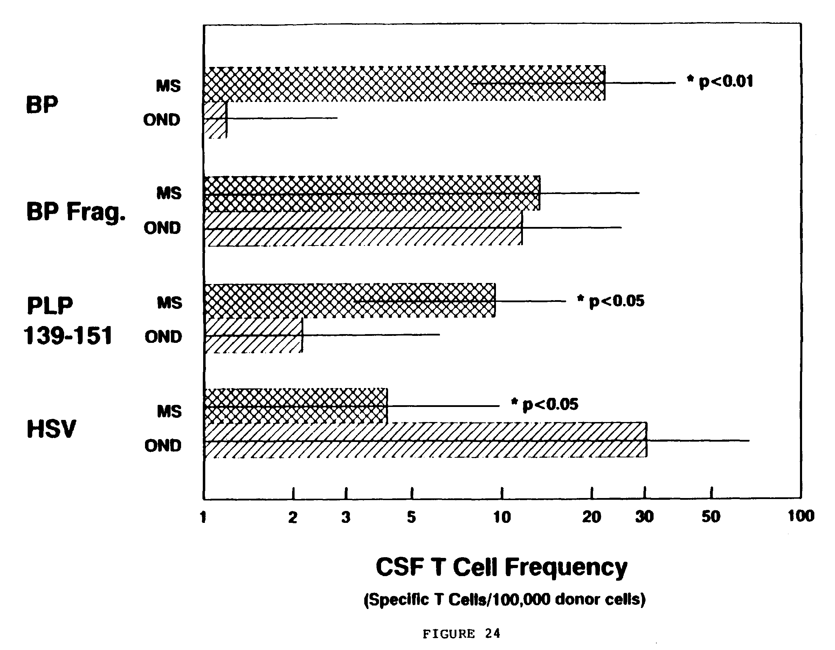

**Figure 24.** Frequency of myelin antigen and HSV reactive T cells in the CSF of MS patients and controls. The frequency of antigen specific T cells from the CSF was calculated by dividing the number of antigen reactive clones recovered from each donor by the total number of CSF T cells analyzed. The values presented represent the mean ± S.D. for the 9 MS patients and 6 OND patients analyzed.

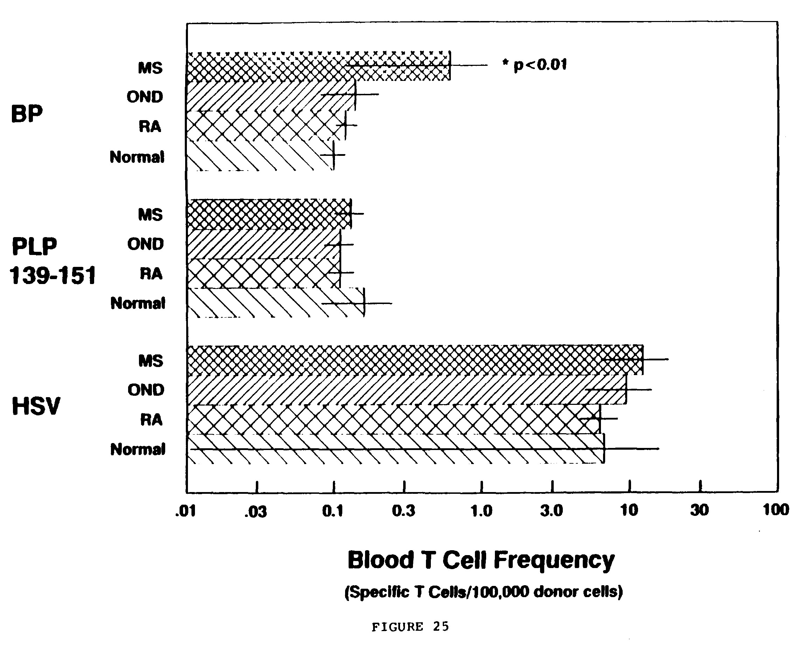

**Figure 25****.** Frequency of myelin antigen and HSV reactive T cells in the blood of MS patients and control. The blood MNC of each patient were separated by Ficoll density gradient centrifugation and subjected to a limiting dilution analysis (Lefkovits *et al., Immun. Today 5*:265-268, 295-298 (1984)), in which the range of cell dilutions tested were 0.31-5.0 x 10⁵ cells per well for BP (50 µg/ml) and PLP₁₃₉₋₁₅₁ (50 µg/ml), and 0.01-0.16 x 10⁵ cells per well for HSV antigen (1/200 of stock solution, Whittaker, Bethesda, MD). Positive proliferation responses to antigens had either a stimulation index greater than 3.0 or a delta CPM of 1,000 over background. The mean percentage of non-responding microcultures of all donors was calculated at each cell dilution, and its negative logarithm was plotted on the y axis and the cell input per well on a linear scale on the x axis. At least two experimental points were used to fit a straight line passing through the origin (20%), representing two interactive cell types. The number of cells containing an average of one precursor cell was determined at the level of 37% non-responding cultures (Lefkovits *et al., Immun. Today 5*:265-268, 295-298 (1984)).

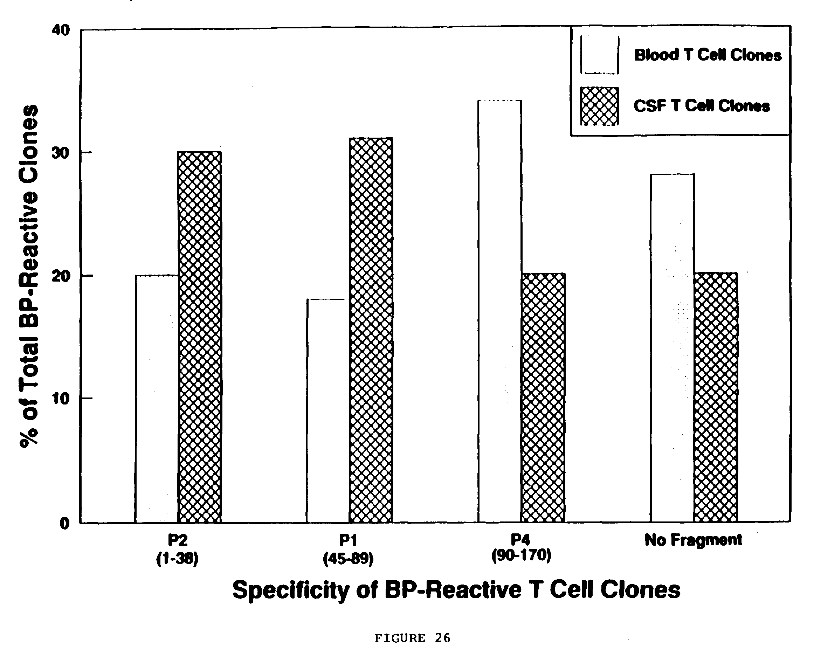

**Figure 26****.** Comparison of peptide specificities of Hu-BP reactive T cell clones in CSF and blood of MS patients. CSF T cells (1000/well) were expanded in IL-2 and IL-4-enriched medium with irradiated (4,500 rad) autologous MNC (1 x 10⁵) without Hu-BP stimulation prior to the assay. Blood T cell clones were obtained by limiting dilution of Hu-BP specific T cell lines that bad been restimulated twice with Hu-BP. The specificity of both CSF and blood T cells was evaluated by thymidine uptake 72 hr after incubating 2 x 104 T cells with 1 x 105 irradiated (4.500 rad) autologous MNC in 0.2 ml triplicate cultures in a round-bottomed 96-well microtiter plate in the absence of antigens and in the presence of 2µg/ml Concanavalin A, 50µg/ml Hu-BP, 50µg/ml Hu-BP fragments: P1, P2, and P4. All clones responded with a stimulation index of > 3.0 or >500 CPM over background. The data represent paired analysis of 71 CSF T cell clones and 50 blood T cell clones from 9 donors.

### DESCRIPTION OF THE PREFERRED EMBODIMENTS

In the following description, reference will be made to various methodologies known to those of skill in the art of immunology, cell biology, and molecular biology.

The peptides and compositions of this invention are applicable to human and veterinary uses.

The peptides of the present invention comprise sequences of about 15-30 amino acids which are immunogenic, that is, capable of inducing an immune response when injected into a subject.

By "functional derivative" is meant a "fragment," "variant," "analog," or "chemical derivative" of the peptide, which terms are defined below.

It is understood that the amino acid sequence comprising the peptide of this invention can be used alone or bound to, or contained within the sequence of, a longer peptide. The longer peptide may include sequences of an unrelated peptide, such as a carrier protein used to enhance the immunogenicity of the TCR oligopeptide. Such carriers are well known in the art and include heterologous proteins such as, for example, keyhole limpet hemocyanin (KLH), bovine serum albumin, tetanus toxoid and the like. Also included within the scope of this invention is the peptide conjugated to an antibody, and the peptide conjugated to a toxin. The toxins of this invention include the ribosomal inhibitory protein, such as, for example, the ricin A chain o*r Pseudomonas* toxin.

As used herein, "marker TCR" refers to a TCR which is characteristic of a specified immune-related disease, such as autoimmune disease or malignant disease (i.e. cancer).

The term "immune-related disease" as used herein refers to a disease in which the immune system is involved in the pathogenesis of the disease, or in which appropriate stimulation of the immune system can result in protection from the disease. A preferred example of an immune-related disease to which this invention is directed is an autoimmune disease. Non-limiting examples of the autoimmune diseases contemplated by this invention are rheumatoid arthritis (RA), myasthenia gravis (MG), multiple sclerosis (MS), systemic lupus erythematosus (SLE), autoimmune thyroiditis (Hashimoto's thyroiditis), Graves' disease, inflammatory bowel disease, autoimmune uveoretinitis, polymyositis and certain types of diabetes.

Thus, a marker TCR for MS is a TCR which is capable of binding the complex between self MHC and the MBP fragment (or the MBP fragment alone), the MBP comprising the major autoantigen characteristic of this disease. In other autoimmune diseases, other TCRs serve as markers, as they are specific for the complex between MHC molecules and the autoantigens involved in these diseases. For example, in myasthenia gravis (MG), the autoantigen is thought to be the nicotinic acetylcholine receptor (AChR). Therefore, an identifiable TCR which binds AChR in the context of self MHC (or directly) and is expressed by AChR-reactive T cells which mediated the disease is a "marker TCR" for MG. Those of skill will recognize that determination of a marker TCR and of immunogenic peptides may be accomplished with the exercise of routine skill, using screening methods as are well-known in the art, when the teachings of the present invention are fully appreciated.

Also intended as immune-related diseases as used herein are malignancies wherein the tumor cell carries a tumor marker, such as a tumor antigen, capable of being recognized and responded to by the immune system. The TCR can serve as a tumor marker on T cell leukemia or T cell lymphoma cells.

In a subjected afflicted with, or susceptible to, an immune-related disease, introduction of the peptide carrying the amino acid sequences of the invention results in generation of an immune response directed to the TCR and protection from the immune-related disease.

By the term "protection" from the disease as used herein is intended "prevention," "suppression" or "treatment" of the disease. "Prevention" involves administration of the protective composition prior to the induction of the disease. Thus, for example, in the animal model, EAE, successful administration of a protective composition prior to injection of the encephalitogen that induces the disease results in "prevention" of the disease.

"Suppression" involves administration of the composition after the inductive event but prior to the clinical appearance of the disease. Again, using the EAE example, successful administration of a protective composition after injection of the encephalitogen, but prior to the appearance of neurological symptoms comprises "suppression" of the disease.

"Treatment" involves administration of the protective composition after the appearance of the disease. In the EAE example, successful administration of a protective composition after injection of the encephalitogen and after clinical signs have developed comprises "treatment" of the disease.

It will be understood that in human medicine, it is not always possible to distinguish between "preventing" and "suppressing" since the ultimate inductive event or events may be unknown, latent, or the patient is not ascertained until well after the occurrence of the event or events. Therefore, it is common to use the term "prophylaxis" as distinct from "treatment" to encompass both "preventing" and "suppressing" as defined herein. The term "protection," as used herein, is meant to include "prophylaxis."

The subject's immune response is directed to the particular TCRs which mark those T cells mediating the autoimmune process, thus inhibiting the function of the pernicious T cells.

A "fragment" of the peptide of the present invention, refers to any subset of the molecule, that is, a shorter peptide.

A "variant" of the peptide refers to a molecule substantially similar to either the entire peptide or a fragment thereof. Variant peptides may be conveniently prepared by direct chemical synthesis of the variant peptide, using methods well-known in the art.

Alternatively, amino acid sequence variants of the peptide can be prepared by mutations in the DNA which encodes the synthesized peptide. Such variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame and preferably will not create complementary regions that could produce secondary mRNA structure (see European Patent Publication No. EP 75,444).

At the genetic level, these variants ordinarily are prepared by site-directed mutagenesis of nucleotides in the DNA encoding the peptide molecule, thereby producing DNA encoding the variant, and thereafter expressing the DNA in recombinant cell culture. The variants typically exhibit the same qualitative biological activity as the nonvariant peptide.

Preparation of a peptide variant in accordance herewith is preferably achieved by site-specific mutagenesis of DNA that encodes an earlier prepared variant or a nonvariant version of the TCR protein or peptide. Site-specific mutagenesis allows the production of peptide variants through the use of specific oligonucleotide sequences that encode the DNA sequence of the desired mutation, as well as a sufficient number of adjacent nucleotides, to provide a primer sequence of sufficient size and sequence complexity to form a stable duplex on both sides of the deletion junction being traversed. The technique of site-specific mutagenesis is well known in the art, as exemplified by Adelman *et al., DNA 2*:183 (1983). Typical vectors useful in site-directed mutagenesis include vectors such as the M13 phage, for example, as disclosed by Messing *et al., Third Cleveland Symposium on Macromolecules and Recombinant DNA*, Walton, A., ed., Elsevier, Amsterdam (1981). These phage are readily commercially available and their use is generally well known to those skilled in the art Alternatively, plasmid vectors that contain a single-stranded phage origin of replication (Veira *et al., Meth. Enzymol. 153*:3 (1987)) may be employed to obtain single-stranded DNA.

In general, site-directed mutagenesis in accordance herewith is performed by first obtaining a single-stranded vector that includes within its sequence a DNA sequence that encodes the relevant peptide. An oligonucleotide primer bearing the desired mutated sequence is prepared, generally synthetically, for example, by the method of Crea *et al., Proc. Natl. Acad. Sci.* (USA) 75:5765 (1978). This primer is then annealed with the single-stranded protein-sequence-containing vector, and subjected to DNA-polymerizing enzymes such as *E. coli* polymerase I Klenow fragment, to complete the synthesis of the mutation-bearing strand. Thus, a mutated sequence and the second strand bears the desired mutation. This heteroduplex vector is then used to transform appropriate cells and clones are selected that include recombinant vectors bearing the mutated sequence arrangement.

The mutated protein region may be removed and placed in an appropriate vector for protein production, generally an expression vector of the type that may be employed for transformation of an appropriate host.

An example of a terminal insertion includes a fusion of a signal sequence, whether heterologous or homologous to the host cell, to the N-terminus of the peptide molecule to facilitate the secretion of mature peptide molecule from recombinant hosts.

Another group of variants is those in which at least one amino acid residue in the peptide molecule, and preferably, only one, has been removed and a different residue inserted in its place. Such substitutions preferably are made in accordance with the following list when it is desired to modulate finely the characteristics of a peptide molecule.

| Original Residue | Exemplary Substitutions | Original Residue | Exemplary Substitutions |
|---|---|---|---|
| Ala | gly; ser | Leu | ile; val |
| Arg | lys | Lys | arg; gln; glu |
| Asn | gly; his | Met | leu; tyr; ile |
| Asp | glu | Phe | met; leu; tyr |
| Cys | ser | Ser | thr |
| Gln | asn | Thr | ser |
| Glu | asp | Trp | tyr |
| Gly | ala; pro | Tyr | trp; phe |
| His | asn; gln | Val | ile; leu |
| Ile | leu; val | | |

Substantial changes in functional or immunological properties are made by selecting substitutions that are less conservative than those in the above list, that is, by selecting residues that differ more significantly in their effect on maintaining (a) the structure of the peptide backbone in the area of the substitution, for example, as a sheet or helical conformation, (b) the charge or hydrophobicity of the molecule at the target site, or (c) the bulk of the side chain. The substitutions that in general are expected are those in which (a) glycine and/or proline is substituted by another amino acid or is deleted or inserted; (b) a hydrophilic residue, e.g., seryl or threonyl, is substituted for (or by) a hydrophobic residue, e.g., leucyl, isoleucyl, phenylalanyl, valyl, or alanyl; (c) a cysteine residue is substituted for (or by) any other residue; (d) a residue having an electropositive side chain, e.g., lysyl, arginyl, or histidyl, is substituted for (or by) a residue having an electronegative charge, e.g., glutamyl or aspartyl; or (e) a residue having a bulky side chain, e.g., phenylalanine, is substituted for (or by) one not having such a side chain, e.g., glycine.

Most deletions and insertions, and substitutions in particular, are not expected to produce radical changes in the characteristics of the peptide molecule. However, when it is difficult to predict the exact effect of the substitution, deletion, or insertion in advance of doing so, one skilled in the art will appreciate that the effect will be evaluated by routine screening assays. For example, a variant typically is made by site-specific mutagenesis of the peptide molecule-encoding nucleic acid, expression of the variant nucleic acid in recombinant cell culture, and, optionally, purification from the cell culture, for example, by immunoaffinity adsorption on an anti-peptide antibody column (to absorb the variant by binding it to at least one epitope).

The activity of the cell lysate or purified peptide variant is then screened in a suitable screening assay for the desired characteristic. For example, a change in the immunological character of the peptide molecule, such as binding to a given antibody, is measured by a competitive type immunoassay. Changes in T cell recognition of the variant peptide are measured by a DH assay *in vivo* or a T cell proliferation assay *in vitro*. Modifications of such peptide properties as redox or thermal stability, hydrophobicity, susceptibility to proteolytic degradation or the tendency to aggregate with carriers or into multimers are assayed by methods well known to the ordinarily skilled artisan.

An "analog" of a peptide refers to a non-natural molecule substantially similar to either the entire molecule or a fragment thereof.

A "chemical derivative" of a peptide of this invention contains additional chemical moieties not normally a part of the peptide. Covalent modifications of the peptides are included within the scope of this invention. Such modifications may be introduced into the molecule by reacting targeted amino acid residues of the peptide with an organic derivatizing agent that is capable of reacting with selected side chains or terminal residues.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylprocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylissurea; 2,4 pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high PKₐ of the guanidine functional group. Furthermore, these reagents may react with the groups of lysine as well as the arginine epsilon-amino group.

The specific modification of tyrosyl residues *per se* has been studied extensively, with particular interest in introducing spectral labels into tyrosyl residues by reaction with aromatic diazonium compounds or tetranitromethane. Most commonly, N-acetylimidizol and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method being suitable.

Carboxyl side groups (aspartyl or glutamyl) are selectively modified by reaction with carbodiimides (R'-N-C-N-R') such as 1-cyclohexyl-3-(2-morpholinyl-(4- ethyl) carbodiimide or 1-ethyl-3 (4 azonia 4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Derivatization with bifunctional agents is useful for cross-linking the peptide to a water-insoluble support matrix or to other macromolecular carriers. Commonly used cross-linking agents include, e.g., 1, 1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophanyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Patent Nos. 3,969,287; 3,691,016; 4,195,128; 4,247,642; 4,229,537; and 4,330,440 are employed for protein immobilization.

Other modifications include hydroxylation of proline and lysine, phosphorylation of hydroxyl groups of seryl or theonyl residues, methylation of the α-amino groups of lysine, arginine, and histidine side chains (T.E. Creighton, *Proteins: Structure and Molecule Properties*, W.H. Freeman & Co., San Francisco, pp. 79-86 (1983)), acetylation of the N-terminal amine, and, in some instances, amidation of the C-terminal carboxyl groups.

Such derivatized moieties may improve the peptide's solubility, absorption, biological half life, and the like. The moieties may alternatively eliminate or attenuate any undesirable side effect of the peptide and the like. Moieties capable of mediating such effects are disclosed, for example, in *Remington's Pharmaceutical Sciences*, 16th ed., Mack Publishing Co., Easton, PA (1980).

### Malignant Disease.

Also susceptible to methods of the invention are lymphomas and leukemias. Lymphomas and many leukemias are tumors made up of lymphocytes that undergo uncontrolled proliferation (e.g., are malignant). Since several classes of leukemia and lymphoma are composed of T cells derived from a single malignant T cell precursor, all of the tumor cells bear the same TCR, which thus serves as a "tumor marker" which can be the target of protective compositions of this invention. Similarly, surface immunoglobulins can serve as tumor markers for B cell leukemias or lymphomas.

One embodiment of the invention is directed to the enhancement of an anti-tumor response by targeting the TCR of those T cells reacting against the "tumor marker" rather than the tumor marker itself. Thus, an immune response directed to the TCR on a tumor-specific T cell can be used to up-regulate the antitumor response for the benefit of the host.

In fact, it will be appreciated that any disease involving a cell having a surface molecule that distinguishes that cell from other cells of the same histological type and from cells of a different histological type, contains a characteristic "marker," and will be susceptible to treatment by compositions which induce an immune response to the "marker," thereby altering activity of the cell bearing the "marker."

According to the present invention, the marker molecule itself may be relatively nonimmunogenic; it requires, at minimum, a characteristic antigenic epitope. This epitope itself may be inherently immunogenic,or it can be rendered immunogenic by treatments well known in the art, such as conjugation to an immunogenic carrier molecule. Thus, an epitope of a marker protein, either as a free peptide or in a form rendering it immunogenic, is capable of eliciting an antibody response, a cell-mediated immune response, or both, as conceived in the invention. Compositions of the present invention incorporate not the entire marker protein, but rather, a specific peptide region which is immunogenic or antigenic.

### Identification of Marker TCR-Bearing T Cells

The present invention utilizes a synthetic peptide that represents a region of the TCR having biological importance in ligand/MHC binding, and that is characteristic of TCR Vβ5. 2 and 6. 2 gene families.

Marker TCRs associated with a given disease are identified using known techniques. A genetic approach using patients known to have MG or MS was described by Oksenberg, *et al., Proc. Natl. Acad. Sci.* USA *86*:988-992 (1989). Sequences of the appropriate TCR β chain have been obtained by genomic analysis using restriction fragment length polymorphisms found in families having a prevalence of the particular autoimmune disease, as described by Seboun, *et al., Cell 87*:1095-1100 (1989); Burns, *et al., J. Exp. Med. 169*:27-39 (1989)).

It thus will be appreciated that, for the purposes of the present invention, determination of the marker TCR associated with an autoimmune disease and identification of peptides comprising an immunogenic sequence do not require that the autoantigen be characterized. It is sufficient that (a) the autoimmune disease involves a T cell-mediated immune response as a necessary part of the pathogenetic process, and (b) the disease has an organ-, tissue- or cell-specific target. In fact, as is known in the art (see, for example, Theofilopoulos, *A., supra*), the autoimmune disease may not involve an autoantigen at all at the inductive stage, but rather, may represent a response to an exogenous antigen,such as a bacterial or viral antigen, which is cross-reactive with self antigens, or results in an immunopathologic response directed to the exogenous antigen present in the host.

T cells recognizing an autoantigen or autoimmune disease-associated antigen (such as certain viral or bacterial antigens) are cloned, and may be fused to an immortalizing cell, such as a long term T cell line, a T cell lymphoma line or a T cell hybridoma, and are grown in culture. The cultured cells serve as the source of cDNA encoding the appropriate TCR. Such cDNA is cloned and expressed by methods well known in the art. (See, for example, Maniatis *et al., Molecular Cloning: A Laboratory Manual* (1982)).

In addition to the foregoing approaches, it will be appreciated that advantage may be taken of animal models to identify the TCR variable region loci which are associated with the autoimmune disease. Animals which are susceptible to any of a number of autoimmune diseases, non-limiting examples of which include EAE, experimental MG, experimental autoimmune thyroiditis, adjuvant arthritis, collagen-induced arthritis, and the like, have a particular TCR variable locus associated with the disease which can be identified *in vitro*.

By the term "susceptible to a disease" is intended a state in which the animal possesses a gene or genes known to be associated with the disease, thereby increasing the risk that the individual with that gene or genes will develop that disease compared to the general population. Genes known to be associated with autoimmune diseases, for example, include MHC genes (especially class II), immunoglobulin V genes, TCR V genes, and the like. The term "susceptible" is also intended to encompass those individuals who actually have the disease. While a complete correlation between an autoimmune disease and the usage of a particular TCR is neither expected nor necessary to successfully practice the present invention, high correlations of about 60-70% have been found for presence or expression of a particular variable region gene and susceptibility to autoimmune disease in animals.

In an alternate embodiment of this invention, T cells isolated from humans who are susceptible to an autoimmune disease, and in particular susceptible individuals who have the autoimmune disease, are expanded in culture. Techniques for T cell expansion are described by Zamvil *et al., Nature 319*:355-358 (1985), and *Nature 324*:258-260 (1986).

In one embodiment employing this method, patient peripheral blood lymphocytes are removed and stimulated with the autoantigen or a specific peptide derived therefrom or related thereto, which is capable of stimulation comparable to that of the autoantigen. The autoantigen (or related peptide) is added to the lymphocyte cultures for several days. In one embodiment, cells are simulated with autoantigen for 5-6 days. In other embodiments, cells are stimulated for longer periods of time. The time required for stimulation is a function of the proportion of reactive cells in the blood sample, the activation state of these cells, and the potency of the stimulating preparation, and is readily determinable by one of skill in the art. After culture under such selective conditions, about 5 x 10⁵ viable cells are isolated and restimulated with about 3 x 10⁷ autologous antigen-presenting cells (irradiated to prevent their proliferation, such as with about 2500-4500 rad) and about 20 µg/ml of the autoantigen (or related peptide). About 7 days later, viable cells are collected and cloned by limiting dilution in the presence of about 10³ - 10⁶ antigen presenting cells, for example about 5 x 10⁵ antigen-presenting cells, and human IL-2 or crude or pure combinations of lymphocyte growth factors (such as, for example, IL-4). The cells of such a T cell line are expanded and grown in tissue culture flasks for about one to two weeks. Such lines can be multiply restimulated with antigen-presenting cells, autoantigen preparations, and IL-2. Restimulation can typically be carried out once a week. If desired, such T cells can be cloned by any of a number of methods known in the art, such as, for example, limiting dilution or by picking cells from colonies growing in soft agar, generally about 2 days after restimulation.

In another embodiment, lymphocytes from an organ or body fluid are first cultured in the presence of IL-2. Under these conditions, selection will occur for cells already activated and only such cells grow. Subsequently, such T cells are stimulated with antigen-presenting cells and an autoantigen preparation. Using this approach, MBP-specific T cells from the spinal cord of rats with EAE can be selectively expanded *in vitro*.

As used in the present invention, the term "autoantigen" is not intended to be limiting to a defined or known macromolecule. For example, in the case of type I diabetes, the particular antigen associated with pancreatic islet (or beta) cells that is the trigger or target antigen of the T cell-mediated autoimmune response is unknown. For the present invention, the autoantigen used to stimulate cells *in vitro*, as described above, can comprise whole pancreatic islet cells, crude membrane preparations derived from such cells, partially purified purified membrane components, or when identified, the diabetogenic autoantigen. The same is true for other autoimmune diseases for which a unique autoantigen has not yet been identified, including Hashimoto's thyroiditis, arthritis in which the autoantigen is not collagen, Sjogren's disease, polymyositis, arteriosclerosis, and the like. One of skill will appreciate that as long as the immunogenic moiety to which the T cells respond is present in the stimulatory preparation, the methods of the invention can be carried out as described.

The presence of autoantigen-specific reactive T cells in the cloned, expanded T cell population can be readily determined by testing the ability of the cells to be activated in the presence of the autoantigen. Many assays are available, and well known in the art, to measure early or late events in the T cell activation process. Example of such methods include, but are not limited to, T cell proliferation (which can be measured as the uptake of radiolabeled thymidine), the secretion of interleukin-2, intracellular calcium mobilization, translocation of particular membrane enzymes involved in inositol phosphate metabolism, and changes in expression of cell surface molecules (which can be determined by flow cytometry).

The TCR expressed by a T cell clone responding to a particular autoantigen can be identified using TCR-specific antibodies, either polyclonal, monoclonal or chimeric (see below) which are specific for a TCR variable segment, to detect surface expression, employing techniques of fluorescence microscopy, flow cytometry, immunocytochemistry, or other techniques known in the art. Such antibodies have been described for a number of TCR α β chain V regions (see, for example, Owhashi *et al., supra;* Gascoigne *et al., supra*; Kappler *et al.*, 1987, 1988 (*supra*); and MacDonald, H.R., *supra*).

Alternatively, the DNA or mRNA of the T cell clone can be probed directly, or after amplification by the polymerase chain reaction (Synha *et al., Science 239*:1026 (1988); Saiki *et al., Nature 324*:163 (1986), by specific hybridization with nucleic acid probes for the various TCR gene families, using hybridization methods well known in the art. The TCR sequence, or a part thereof, can then be obtained directly from the amplified, rearranged DNA or mRNA.

Expression of a particular TCR can also be identified by determining the nucleic acid sequence encoding at least part of the TCR, for example, after cloning the TCR V gene, or by determining the amino acid sequence of at least part of a TCR protein. It will be apparent that any of the abovementioned approaches, or additional approaches known to one of skill in the art, will result in the identifying of the TCR expressed on a T cell or clone or line of T cells. This information is needed for the selection of an amino acid sequence which comprises the peptide or pharmaceutical preparations of this invention.

Where no specific autoantigen has been identified, the oligoclonality of T cells in the anatomic region associated with the disease can be used as a basis for enrichment of reactive T cells. For instance, cells uniquely associated with rheumatoid arthritis are found in the synovial fluid of the joint; cells uniquely associated with MS are found in the cerebrospinal fluid (CSF); and disease-associated T cells infiltrate the thyroid tissue in Hashimoto's thyroiditis and in Graves' disease. In these instances, T cells are isolated from the relevant anatomical location, and the cells expanded in culture as described above. (See also, Londei *et al., Science 228*:85-89 (1985); Londei *et al., Acta Endocrinol. 115* (suppl. 281):86-89 (1987); Stamenkovic *et al. Proc. Natl. Acad. Sci.* USA *85*:1179-1183 (1988); Lipoldova *et al. J. Autoimmun. 2*:1-13 (1989); Oksenberg *et al., supra*). The DNA or mRNA of such cells is isolated, cDNA prepared, and the differences in sequences of cDNA encoding the variable TCR loci are established by comparison of afflicted with unafflicted subjects. As an alternative to expanding the cells in culture, cellular DNA or, preferably, cDNA made from mRNA, can be obtained directly from T cells isolated from the subject, and the nucleic acid expanded by the PCR reaction, as above.

The antigens associated with a number of human and animal model autoimmune diseases are presently known. Type II collagen and *Mycobacterium tuberculosis* 65 kD heat shock protein are antigens associated with rheumatoid arthritis; AChR is associated with MG, and with experimental allergic myasthenia gravis (EAMG) which can be induced in mice. Thyroglobulin is known to be the antigen associated with experimental allergic thyroiditis (EAT) in mouse. A similar disease, Hashimoto's thyroiditis involves an immune response to an antigen on thyroid follicular cells. In Graves' disease, the immune response is directed to the thyrotropin receptor on thyroid cells. Myelin basic protein (MBP) and proteolipid protein (PLP) are known to be associated with experimental allergic encephalomyelitis (EAE) in mouse and rat. EAE is a recognized model for multiple sclerosis in humans.

Therefore, those of skill will appreciate that the present invention is directed in one aspect to identification of peptides useful for prevention or therapy of human and animal diseases, including but not limited to those mentioned above.

### Selection of Antigenic Peptides

An important embodiment of this invention comprises the combined method of identifying a TCR associated with an autoimmune disease, determining which oligopeptide sequence of the TCR is both immunogenic and important for T cell action in the disease process, synthesizing that peptide, and using it as a therapeutic agent.

Regions of relevant TCR sequences are identified for synthesis on the basis of their predicted antigenic or immunogenic properties. By the term "immunogenic" is intended the capacity of a peptide to induce an immune response, either T cell-mediated, antibody, or both. By the term "antigenic" is intended the capability of a peptide to be recognized, in free form by antibodies and in the context of MHC molecules in the case of antigen-specific T cells. Regions of a protein or peptide that are likely to be immunogenic or antigenic for T cells are identified, for example, using the approaches and algorithms described by Margalit *et al. (J. Immunol.* 138:2213-2229 (1987) and Rothbard *et al.* EMBO J.7:93-100 (1988)). The Margalit *et al.* approach is based on analysis of immunodominant helper T cell antigenic sites leading to development of an algorithm based on an amphipathic helix model, in which antigenic sites are postulated to be helices with one predominantly polar and one predominantly apolar face. The approach of Rothbard *et al.* recognizes motifs similar to epitopes recognized preferentially by T helper or T cytotoxic cell clones, which can predict accurately areas within protein sequences that are capable of being recognized by MHC class I and II molecules, such recognition being assumed as necessary for T cell immunogenicity and antigenicity.

The regions of the TCR which are of immunoregulatory importance for the purposes of this invention are human Vβ5.2 (26-43), Vβ5.2 (39-59), Vβ5.2 (59-79), human Vβ6.1 (1-22), Vβ6.1 (39-59) and Vβ6.1 (70-88).

The use of the above approach to select peptide sequences for use in treating MS in human patients and EAE in rats exemplifies the success of this approach. For example, a peptide comprising 16 amino acids corresponding to CDR1 of the marker TCR for EAE in Lewis rats, Vβ8 (25-41) was predicted by the above algorithms not to be immunogenic for T cells. In fact, this peptide does not induce T cell immunity and does not protect Lewis rats from EAE. A peptide corresponding to the CDR1 of a different TCR β chain which is not associated with EAE, Vβ 14(25-41), was predicted to be immunogenic for T cells, and indeed was found to induce T cell immunity in Lewis rats, but, as expected, did not protect from EAE. Similarly the CDR2 peptide, Vβ 14(39-59), corresponding to an TCR not associated with EAE, was predicted to be immunogenic, and did induce immunity, but, again, did not provide protection from EAE. According to the invention, the CDR2-related peptide of the relevant TCR, Vβ8(39-59), was predicted to be both immunogenic and protective in EAE, and indeed, was shown to be so (see Examples, below).

The size of the peptide selected for use in this invention is largely determined by the requirement for immunogenicity, while maintaining the minimal epitope structure such that a T cell or antibody specific for the peptide will recognize and react with the TCR on an intact T cell. For example, peptides of this invention, in order to be sufficiently immunogenic and to have a high probability of including the relevant epitope of the TCR which can lead to modulation of T cell activity, are of the range of about 15-30 amino acids, although peptides of differing length are also contemplated. The successful use of a 21 amino acid TCR peptide present on the TCR β chain associated with EAE in rats to treat EAE according to the methods of this invention is amply demonstrated in the Examples below.

Immunogenicity of peptides useful in the present invention can be screened by well-known methods, such as use of a DH response in an animal. In such a response, an animal is "sensitized" by injection of an appropriate dose of an antigen, typically subcutaneously (SC), frequently with an adjuvant, such as complete Freund's adjuvant (CFA). Generally about 5-15 days later, the response is "elicited" by challenging the animal, typically intradermally (ID), with an appropriate dose of the antigen, typically in saline or other buffer. The response is assessed 24-48 hours later. Non-limiting examples of assay methods which measure DH include size of erythema (redness) and induration (swelling) at the site of antigen injection, ear swelling, footpad swelling, tail swelling, accumulation of systemically injected ¹²⁵I-labeled iododeoxyuridine in the challenge site, accumulation of intravenously (IV) injected radiolabeled serum protein, such as albumin, in the challenge site, and accumulation of IV-injected labeled inflammatory cells, such as lymphocytes or neutrophils, in the challenge, site. For example, an ear swelling response upon appropriate ID challenge in the ear pinna of about 0.15-0.25 mm, and preferably about 0.20 mm (in a Lewis rat) represents a positive DH response. One skilled in the art will understand that variations in peptide size, dose, route of sensitization or elicitation of DH, carriers used, adjuvants used, etc., will affect the timing and extent of the DH response.

For a peptide to be considered immunogenic, as intended here, a dose of about 10-200 µg per animal, and preferably about 25-100 µg of the peptide per animal, should be able to sensitize an animal for a DH response. Furthermore, in a sensitized animal, a dose of about 1-100 µg, and preferably about 5-50 µg, of the peptide is able to elicit a DH response upon ID challenge.

### Synthesis of Peptides and Assay

The desired peptides, with sequences determined as described above, are prepared using standard synthesis techniques including solution and solid phase sequential amino acid conjugation and recombinant production in prokaryotic or eukaryotic hosts. Verification that the peptide prepared is immunogenic can easily be determined using a DH reaction in an animal (e.g., mouse or rat), as described above. The peptide is administered subcutaneously, and the animal is challenged about 9-14 days later ID in the ear pinna. The ear swelling response, measured 24 or 48 hours after challenge, serves as a simple and reliable measure of the presence of T cell-mediated immunity to the peptide.

Verification of the ability of the immunogenic peptide to actually modulate autoimmunity may be attained using an appropriate animal model, taking into account the species differences in the TCR-related peptides. For example, although it is preferred to use a sequence representing the CDR2 of a human marker TCR in treating humans, the corresponding region of the marker TCR for the animal disease model is used in the animal disease.

Animal model systems which can be used to screen the effectiveness of the peptides in protecting against or treating the disease are available, as discussed above. Of course, the identical peptides may not be effective in humans since they may not correspond to an appropriate site of the disease-associated human TCR, or may not be sufficiently immunogenic in humans. It is to be understood that modifications of the peptide sequence delineated in a particular animal model may be required in order to treat subjects belonging to other species, including humans. Thus, verification that, for example, a particular CDR2-associated peptide sequence is effective in protecting against a particular disease can be obtained in these models, leading to predictions that the corresponding human sequence would be a preferred candidate as an effective peptide therapeutic for humans. Determination of the corresponding TCR sequence in the human (or in different non-human animal species), using approaches described above, thus permits modification of the peptide for use in the human (or other species).

The following is a non-exclusive list of animal disease models of human autoimmune diseases with which a TCR peptide can be assessed for its ability to modify disease, and to induce antibodies and T cells which are capable, upon transfer, of modifying disease. Systemic lupus erythematosus (SLE) is tested in susceptible mice as disclosed by Knight *et al., J. Exp. Med. 147*:1653 (1978) and Reinertsen *et al., N. Eng. J. Med. 299*:515 (1978). MG is tested in SJL/J female mice by inducing the disease with soluble AChR protein from another species as described in Lindstrom *et al., Adv. Immunol. 42*:233-284 (1988). Arthritis is induced in a susceptible strain of mice by injection of Type II collagen as described by Stuart *et al., Ann. Rev. Immunol. 2*:199-218 (1984). Adjuvant arthritis is induced in susceptible rats by injection of Mycobacterial heat shock protein as described by Van Eden *et al., Nature 331*:171-173 (1988). Thyroiditis is induced in mice by administration of thyroglobulin as described by Maron *et al., J. Exp. Med. 152*:1115-1120 (1980). Insulin-dependent diabetes mellitus (IDDM) occurs naturally or can be induced in certain strains of mice such as those described by Kanasawa *et al.*, *Diabetologia 27*:113 (1984). Other mouse strains can be caused to exhibit this disease by transferring lymphocytes from this strain.

EAE in mouse and rat serves as a model for MS in humans. In this model, the demyelinating disease is induced by administration of myelin basic protein (MBP) or proteolipid protein (PLP), or Theiler's virus, as described by Paterson, P.Y., *Textbook of Immunopathology* (Mischer *et al.*, eds.), Grune and Stratton, New York, pp. 179-213 (1986); McFarlin, D.E., *et al., Science 179:*478-480 (1973); and Satoh, J., *et al., J. Immunol. 138*:179-184 (1987).

For measuring preventative, suppressive, or therapeutic benefit of the compositions of this invention in humans, certain clinical outcome measures are used. In MS, for example, quantitative parameters include: (a) clinical disability, (b) on study exacerbation rate, and (c) magnetic resonance imaging (MRI) brain plaque load (which is an important recent parameter used to evaluate MS patients). These measures involve separate blinded examination or unblinded examination by a treating physician. Neuropsychological measures of cognitive impairment are used as an independent determinant of disability. Clinical disability is typically measured by the McAlpine Scale, the Kurtzke Score, a modification Kurtzke Score termed the Expanded Disability Status Score (EDSS). An improvement of _ unit on the EDSS (Range of 1-9) is considered significant. One clinical measure, the patient's ability to walk, is rated by the Ambulation Index, wherein an improvement of 1 or more units is considered significant. These clinical measures are well known in the art and are described in detail in McAlpine et al., Multiple Sclerosis, Livingston Press, Edinburgh (1955); Binken, P.J. et al., Handbook of Clinical Neurology, Volume 9, Amsterdam-North Holland Publishers, Amsterdam (1970); and Field, E.J. et al., Multiple Sclerosis: A Critical Review, M.M.T.P Press, Ltd., Lancaster, England, (1977).

Measurement of improvement in RA, for example, is based on a number of primary clinical endpoints, including resolution or reduction of swelling, reduction in duration of morning stiffness, decreased erythrocyte sedimentation rate and/or C-reactive protein, resolution of rheumatoid-associated conditions as rheumatoid nodules, and reduction in lymphocyte counts. Secondary endpoints include reduction in fatigue and improvement in overall condition as assessed by the patient and the physician. Clinical outcomes are divided into the following: (a) Complete Response - greater than 90% decrease in joint swelling, tenderness, and morning stiffness; (b) Marked Response - 50-90% decrease in joint swelling, tenderness, and morning stiffness; (c) Moderate Response - 30-50% decrease in joint swelling, tenderness, and morning stiffness; and (d) No Response - < 30% decrease in joint swelling, tenderness, and morning stiffness.

Similar measurements which allow evaluation of the preventive, suppressive, or treatment effects of the peptides, antibodies, T cells and other compositions of the present invention in additional immune-related disease are known to those of skill in the art.

### Formulation of Peptides

The preclinical and clinical therapeutic use of the present invention in the treatment of disease or disorders will be best accomplished by those of skill, employing accepted principles of diagnosis and treatment. Such principles are known in the art, and are set forth, for example, in Braunwald *et al.*, eds., *Harrison's Principles of Internal Medicine*, 11th Ed., McGraw-Hill, publisher, New York, N.Y. (1987).

The peptides and compositions of the present invention, are well suited for the preparation of pharmaceutical compositions. The pharmaceutical compositions of the invention may be administered to any animal which may experience the beneficial effects of the compositions of the invention. Foremost among such animals are humans, although the invention is not intended to be so limited.

The pharmaceutical compositions of the present invention may be administered by any means that achieve their intended purpose. For example, administration may be by parenteral, subcutaneous, intravenous, intradermal, intramuscular, intraperitoneal, transdermal, or buccal routes. Alternatively, or concurrently, administration say be by the oral route. The peptides and pharmaceutical compositions can be administered parenterally by bolus injection or by gradual perfusion over time.

The dosage administered will be dependent upon the age, sex, health, and weight of the recipient, kind of concurrent treatment, if any, frequency of treatment, and the nature of the effect desired. The dose ranges for the administration of the compositions of the present invention are those large enough to produce the desired effect, whereby, for example, an immune response to the peptide, as measured by DH or antibody production, is achieved, and the immune-related disease is significantly prevented, suppressed, or treated. The doses should not be so large as to cause adverse side effects, such as unwanted cross reactions, generalized immunosuppression, anaphylactic reactions and the like.

Preferred doses for humans range between about 0.001 - 25 mg/kg body weight.

In addition to peptides of the invention which themselves are pharmacologically active, pharmaceutical compositions may contain suitable pharmaceutically acceptable carriers comprising excipients and auxiliaries which facilitate processing of the active compounds into preparations which can be used pharmaceutically. Preferred compositions include the inclusion of an adjuvant, such as alum, or other adjuvants known in the art. (See, for example, Warren et al., Ann. Rev. Immunol. 4:369-388 (1986); Chedid, L., Feder. Proc. 45:2531-2560 (1986)).

To enhance delivery or bioactivity, the peptides can be incorporated into liposomes using methods and compounds known in the art.

Preparations which can be administered orally in the form of tablets and capsules, preparations which can be administered rectally, such as suppositories, and preparations in the form of solutions for injection or oral introduction, contain from about 0.001 to about 99 percent, preferably from about 0.01 to about 95 percent of active compound(s), together with the excipient.

Suitable excipients are, in particular, fillers such as saccharides, for example lactose or sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

Possible pharmaceutical preparations which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous solutions of the peptides in water-soluble form, for example, water-soluble salts. In addition, suspensions of the peptides as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol, and/or dextran. Optionally, the suspension may also contain stabilizers.

The peptides are formulated using conventional pharmaceutically acceptable parenteral vehicles for administration by injection. These vehicles are nontoxic and therapeutic, and a number of formulations are set forth in *Remington's Pharmaceutical Sciences, (supra)*. Nonlimiting examples of excipients are water, saline, Ringer's solution, dextrose solution and Hank's balanced salt solution. Formulations according to the invention may also contain minor amounts of additives such as substances that maintain isotonicity, physiological pH, and stability.

The peptides of the invention are preferably formulated in purified form substantially free of aggregates and other protein materials, preferably at concentrations of about 1.0 ng/ml to 100 mg/ml.

Effective doses of the peptides of this invention for use in preventing, suppressing, or treating an immune-related disease are in the range of about 1 ng to 100 mg/kg body weight. A preferred dose range is between about 10 ng and 10 mg/kg. A more preferred dose range is between about 100 ng and 1 mg/kg.

The immunogenicity of the peptide may be enhanced by including it in a longer peptide or chain or by conjugating it to "immunological' carriers, such as KLH, serum albumin, tetanus toxoid, and the like, using standard linking techniques. A variety of such methods is known in the art, e.g., use of condensing agents such as dicyclohexylcarbodiimide or use of linkers, such as those commercially available from Pierce Chemical Co., Rockford, IL.

For the passive immunization with the TCR peptide-specific T cell preparations of this invention, the harvested T cells are suspended in a suitable vehicle, such as physiologically buffered saline, and injected into the subject in an amount of approximately 10⁵-10⁹ cells per injection. Doses of TCR peptide-specific antibodies vary as a function of antibody species origin, isotype, affinity, nature (polyclonal, monoclonal, chimeric) and other characteristics which are known to one of skill. For example, a monoclonal antibody to a TCR peptide will be administered at a dose of between 0.01 - 50 mg/kg.

Also contemplated within the scope of this invention is passive immunization with a combination of protective T cells and TCR peptide-specific antibodies (polyclonal, monoclonal, or chimeric) in free or conjugated form.

The following examples are intended to be illustrative, but not to limit, the invention.

### EXAMPLE I

### Treatment of Human MS Patients With TCR Peptides

Currently, no effective treatment for MS is known. (Harrison's Principles of Internal Medicine, 12th ed. Wilson et al., McGraw Hill, Inc. 1991) Therapeutic efforts are directed toward amelioration of the acute episode, prevention of relapses or progression of the disease, and relief of symptoms. The clinical manifestations of MS depend upon which nerve group or region of the brainstem, cerebellar or spinal cord is involved. Spinal cord involvement is the predominating feature in most advanced cases of MS.

In acute episodes of disease, glucocorticoid treatment has been suggested as having the potential to lessen the severity of symptoms and speed recovery, however, even its proponents point out that ultimate recovery is not improved by this drug nor is the extent of permanent disability altered. ACTH is the preferred glucocorticoid of clinicians since the only controlled trials which demonstrated any efficacy of glucocorticoid therapy in episodes of MS and optic neuritis were performed with this drug. However, use of long term steroids is not advised.

Immunosuppressive agents such as azathioprine and cyclophosphamide have been claimed to reduce the number of relapses in several series, but there is no consensus about the efficacy of these drugs either.

The current recommendations for the treatment of MS revolve around attempting to avoid exacerbation of the symptoms. Patients are advised to avoid excess fatigue and extremes of temperature and eat a balanced diet. (The above discussion is primarily from Chapter 356 of Harrison's Principles of Internal Medicine, 12th ed. 1991.)

The above discussion clearly points out that there exists a desperate need for a clinically efficacious treatment for MS.

The present example provides the first demonstration of a therapy resulting in clinical improvement of MS patients. The clinical data presented are derived from two patients treated in vivo with TCR peptides. The results demonstrate the ability of the Vβ5.2 and Vβ6. 1 peptides to suppress the autoimmune response to myelin basic protein (BP) without concurrent suppression of the immune system response. Most importantly, the data indicate the clinical efficacy of treating MS patients with TCR peptides.

### Methods

Identification of TCR V Genes Used by BP-Specific T Cells. Blood T cells from MS patients and controls were selected for response to human BP. The responder T cell lines were cloned, and each BP-reactive clone was analyzed for the expression of TCR Vα and Vβ genes. In the MS patients studied, the inventor found a preferential utilization of TCR Vβ5.2 and Vβ6.1, whereas in one control donor 11 of 13 clones utilized Vβ14.

From the data, the inventor predicted that each patient would preferentially utilize a limited number (1 or 2) of Vβ genes in response to BP.

Selection of the Antigenic Peptides. The selection of the peptides used in this example was carried out according to the procedure and algorithm described above. Specifically, the regions of relevant TCR sequences were identified for synthesis on the basis of their predicted antigenic or immunogenic properties and/or their occurrence in the CDR2 region. Regions of the protein or peptide that were likely to be immunogenic or antigenic for T cells were identified, using the approaches and algorithms as described herein.

The above approach was used to select the peptide sequences useful for treating multiple sclerosis in humans. The size of the peptide selected for use in this example was largely determined by the requirement for immunogenicity, while maintaining the minimal epitope structure, such that a T cell or antibody specific for the peptide would recognize and react with the TCR on an intact T cell. Thus, it was important to identify antigenic regions of the marker TCR V gene sequence to be used for treatment. A number of sequences of peptides from human TCR Vβ genes were considered for this study. (TCR Vβ5.2 peptides: Vβ-26-43, Vβ5.2-39-59, Vβ5.2-59-78. TCR Vβ6.1 peptides: Vβ6.1-1-22, Vβ6.1-39-59, Vβ6.1-70-88.) The peptides selected were Vβ5.2(39-59) and Vβ6.1(39-59), on the basis of their increased recognition by T cells from MS patients.

The use of the above approach to select peptide sequences for use in treating MS patients exemplifies the success of this approach as described herein. For example, the peptides Vβ5.2(39-59 and Vβ6.1(39-59) were predicted by the above algorithms to be immunogenic for T cells. In fact, as described below, these peptides did produce T cell immunity and appear to be protecting the MS patients from autoimmune reactions to BP.

Treatment with TCR Peptides. The antigenicity of the selected peptide was assayed by determining whether there was a detectable frequency of response from the patient's T cells. Specifically, the differences in frequencies of T cells responding to the various peptides were assayed. The peptide eliciting the strongest frequency of response was then used.

Each patient was treated i.d. with 100ug of TCR peptide in saline, once per week for 4 weeks. The patients were evaluated weekly for evidence of increased response to the peptides, measured by blood T cell frequency analyses in vitro and DTH response. Responses to the peptides were maintained by periodic booster injections of the peptides as needed. Additionally, the patients were evaluated every three months clinically for changes in ambulation index and Kurtzke score.

Parameters Measured. T cell responses to the TCR peptides Vβ5.2 and Vβ6.1, basic protein (BP) and HSV (Herpes simplex virus--used as a recall antigen) were evaluated by lymphocyte proliferation. Patient J.M. was treated with Vβ6.1, and T cell frequencies followed in response to both Vβ6.1 as well as to Vβ5.2 not given as therapy. Patient M.R. was treated with Vβ5.2 and was tested for responses to both Vβ5.2 and Vβ6.1.

Both patients were assessed for their response to BP due to the evidence that the frequency of BP or PLP reactive T cells is higher in MS patients than in normal, neurologic, or autoimmune controls (Allegretta et al., Science 247:718 (1990); Link et al., Neurology40:283 (1990); and Ota et al., Nature 346:183 (1990). The selective immunoregulation strategy offered by the TCR peptides of this invention, as used in this example, provides the first test for the hypothesis that myelin basic protein is a relevant autoantigen in MS.

Immune response to HSV was followed so as to determine whether the TCR therapy was suppressing the overall immune response of the patients. Based on the inventor's experimental model, the patients would show an increased response to the TCR peptide administered. This increased response to the TCR peptide would be coupled to a decreased response to BP. Additionally, the patients would not show a decreased response to HSV or to the other TCR peptide not used as therapy.

Results. The data obtained clearly demonstrate the utility of TCR therapy in MS patients. The data, which measure the frequency of antigen-reactive T cells may be summarized as follows: Patient J.M. (receiving TCR Vβ6.1) had a four fold increase in T cell response to Vβ6.1 (at 1 week post treatment), which tapered off to about a two fold increase (at week 4) vs pre-treatment). The myelin basic protein (BP) cell response was virtually undetectable by week 4 (below the limits of the assay). The response to Vβ5.2 stayed about the same (as expected, since this patient never received the Vβ5.2 peptide), whereas the response to HSV increased over the 4 weeks.

The data from patient M.R. (who received a Vβ5.2 TCR peptide) demonstrates a 30-fold increase in the Vβ5.2 reactive (regulatory) T cells, with a virtual disappearance of the BP reactive T cells by week 4. M.R.'s responses to Vβ6.1 (this patient never received this peptide) and HSV stayed about the same (Figures 12-14).

In addition to the demonstrated increase in the frequency of antigen-reactive T cells, patient J.M. also experienced a dramatic improvement in ambulation, while patient M.R. reports an increase in stamina (able to walk 400 yards without collapsing from exhaustion).

Prior to treatment with TCR peptides, patient J.M.'s ambulation had been progressively deteriorating. For a timed walk of 25 feet, J.M.'s time for walking this distance had progressed from 7 secs. to 12 secs. and then to 29 seconds.

Three months subsequent to treatment with the TCR peptide, J.M. demonstrated an improvement in ambulation. Not only was the deterioration arrested, but ambulation time was dramatically improved from 29 seconds to 21 seconds.

Discussion. The data demonstrate the dramatic ability of the TCR therapy to regulate the immune response to BP, thus providing a method of treatment for MS. The administration of Vβ5.2 and Vβ6.1 showed an increase in T cell response in MS patients. The corresponding decrease in T cell response to BP shows the positive therapeutic effect of the TCR peptide therapy on MS patients. Additionally, the absence of a decrease in the patients' response to HSV demonstrate that the immune system is not compromised by the TCR peptide therapy.

Although the above example utilizes a single peptide for each patient, the invention contemplates the use of a "cocktail' approach in treating patients. The cocktail approach could be useful in patients with clones demonstrating reactivity to more than one V gene region: therefore the cocktail administered to the patient could involve more than one antigenic peptide such as was used in the SJL mouse experiment (Figure 15, Example X).

### EXAMPLE II

### Preferential Cell Receptor Vβ Gene Usage in Myelin Basic Protein Reactive T Cell Clones from Patients with Multiple Sclerosis

### Introduction

Multiple sclerosis (MS) is an autoimmune disease in which T lymphocytes reactive to myelin basic protein (BP) could play a central role. According, the utility of the present invention was demonstrated by cloning T cells specific for BP from the blood of MS patients and normal individuals; followed by the analysis and expression of T cell receptor (TCR) variable (V) region genes. A remarkable bias for usage of Vβ 5.2 and to a lesser extent Vβ6.1 was observed among BP-specific clones from patients but not from controls. Shared Vβ5.2 and Vβ6.1 suggests that the BP-specific clones derived from blood may be relevant to disease pathogenesis. These findings have important implications for the treatment of MS. This example analyzes the TCR Vα and Vβ genes expressed in BP-specific T cells selected from the blood of MS patients and normal individuals.

T cell clones specific for BP and other antigens were derived from peripheral blood mononuclear cells (PBMC) of MS patients and normal individuals, and characterized in terms of response to different BP regions. TCR Vβ and Vα gene expression in these clones was determined using PCR amplification (Oksenberg et al., Nature 345:344-346 (1990); Choi et al., Proc. Natl. Acad. Sci. USA 86:8941-8945 (1989)). A representative example of detected Vβ and Vα expression is shown in Figures 10A and 10B, respectively. Although referred to as Vβ5.2 in Figure 10A, the Vβ5.2 and Vβ5.3 gene products cannot be distinguished since a sequence common to these V genes was used as primer. The clone analyzed in Figure 10 was also shown to express cell surface TCR VP5.2/5.3 antigens by immunofluorescence analysis (see Table 5).

A detailed analysis of clones derived from three MS patients is shown in Table 7. BP-specific clones from patient NL showed a striking preferential usage of Vβ5.2. Surprisingly, specificity for different BP determinants appeared to be independent of this biased TCR Vβ gene usage. For example, clones specific for peptides 45-89 and 1-38, and BP-specific clones that were not stimulated by any of these peptides rearranged Vβ5.2 genes preferentially. Although Vα usage was determined for only four NL clones, the data suggest that Vα may be a major contributor to BP peptide specificity. Thus, Vα8 gene expression was identified in three clones specific for BP peptide 45-89 whereas Vα2 was rearranged in the one clone specific for peptide 1-38. With this limited analysis, however, a major contribution from α to β chain junctional regions (Jα. Dβ. Jβ) cannot be excluded. Despite common usage of Vβ5.2, many of the NL clones appear to be clonally unrelated, separated on the basis of peptide specificity, Vα expression, and minor Vβ gene expression. The clonal relatedness of certain sets, for example the seven Vβ5.2 + clones in which no peptide specificity was apparent, is currently unknown. Determining Vα expression and/or sequencing junctional regions would be the most straightforward way to answer this question. Four of 10 BP-specific clones derived from patient MR also utilized Vβ5.2. As in patient NL, clones with three distinct BP specificities used this particular Vβ gene. Three of six clones from WS also expressed Vβ5.2 genes, of which two utilized the same Vα and had the same specificity. In contrast to the frequent isolation of Vβ5.2+ clones that were BP specific, none of the clones reactive to other antigens or clones that were nonspecific utilized Vβ5.2 (Table 7).

It should be emphasized that great care was taken to avoid contamination of samples with amplified PCR products, and to exclude it as a contributor to the findings. Thus, in all cases, controls included negative reactions with Vβ5.2 and Vβ6.1-specific oligomers but with no RNA template. Whenever possible, freshly prepared RNA was also used to confirm an initially positive Vβ5.2+ clone. Furthermore, clones with different specificities and from different patients as well as from normal individuals were studied at similar times, and preferential Vβ5.2 usage was only detected in BP-specific clones from MS patients (see Tables 7 and 8). Finally, clones from patient MR were analyzed for surface expression of Vβ5.2 determining using a monoclonal anti-Vβ5.2 antibody and immunofluorescence analysis, and complete concordance between gene expression by PCR and cell surface expression by monoclonal antibody was observed.

A striking preferential usage of Vβ5.2 and to a lesser extent Vβ6.1 was apparent in BP-specific clones from seven patients but not from six normal donors (Table 8). Of 48 BP specific clones from MS patients analyzed by PCR for Vβ expression, 27 utilized Vβ5.2 and 0 of 15 non-BP-specific clones were Vβ5.2+ (p<0.001 by Fisher's exact test). In contrast only two of 41 BP-specific clones from normal individuals were Vβ5.2+ (p<0.0001 by Chi-square analysis). The statistical significance of these comparisons is still evident if clones from patient NL are excluded, and the frequency of Vβ5.2+ BP-specific clones in patients is considered to be 14 of 34 (p<0.001 for comparison with BP-specific clones for normals or for comparison with non-BP-specific clones. In regards to the comparison of clones from patients and controls, six of the seven patients studied and only two of the six controls carried HLA-DR2, which is expected from the known frequencies of DR2 in the two populations. Still, only one of ten BP-specific clones from the DR2+ controls was Vβ5.2+ (p<0.01 compared to frequency in MS patients). The 54% frequency in patients is also much greater than the expected peripheral blood level (2.5-5.0%) (Choi et al., Proc. Natl. Acad. Sci. USA 86:8941-8945 (1989); Kappler etal., Science 244:811-813 (1989)). Although overall frequencies of Vβ6.1+BP-specific clones from patients compared with that from controls or compared with non-BP specific clones are not statistically different, the frequency (20%) is still much greater than that expected by chance (peripheral blood percentage predicted by PCR is 5-10%, (Choi et al., Proc. Natl. Acad. Sci. USA 86:8941 (1989)). Furthermore, if only clones that do not express Vβ5.2 are considered, the frequency of Vβ6.1 expression in BP-specific clones from patients compared with controls is statistically different (p<0.02).

Despite the absence of Vβ5.2+ clones, preferential Vβ usage was noted for BP-specific clones derived from normal individuals (Table 8). In one individual, 11 of 13 clones expressed Vβ14, and in two other Vβ7 predominated. No relationship with serologically-determined DR expression was discernable.

Figure 11 shows a summary of Vα utilization in BP-specific clones from patients and controls. In contrast to Vβ usage, BP-specific clones from patients showed much more generalized Vα usage. Vα1,2,7,8, and 10 families were well-represented among the 30 clones analyzed. Because of an initial emphasis on Vβ, it should be noted that only 4 clones from patient NL were analyzed for Vα expression. Perhaps the analysis of more clones might disclose more prominent Vα skewing. Surprisingly, preferential Vα usage was much more apparent in BP-specific clones among controls. Of 22 clones analyzed, 12 expressed Vα2 and eight expressed Vα 15.

In the present report, peripheral blood cells were expanded initially in bulk culture by stimulation with intact human BP, prior to cloning by limiting dilution. Responses to BP peptide 84-102 which dominated the response in the Wucherpfennig study (Wucherpfennig et al., Science 248:1016-1019 (1990)), were notably rare in the current study. In addition, preferential Vβ5.2 expression in the current example appeared to be independent of BP-epitope specificity. Interestingly, this is similar to the preferential Vβ5.2 expression that has been found in mice and rats (Heber-Katz et al., Immunol. Today 10:164-169 (1989)).

Oksenberg etal., (Nature345:344 (1990) and manuscript submitted) used the PCR to analyze directly expression of TCR Vα and Vβ genes in brain samples from MS patients. Limited heterogeneity of both Vα and Vβ transcripts was observed. Interestingly, of nine HLA-DR2 patients, including eight with different DR2 (DW2) molecular phenotypes, eight demonstrated rearranged Vβ5.2 gene and seven demonstrated rearranged Vβ6.1 at the site of brain disease. The correlation with the findings of preferential Vβ5.2 and Vβ6.1 usage in BP-specific clones derived from the peripheral blood of patients in this example is remarkable. Together, the studies provide additional evidence suggesting that peripheral blood T cells are a reflection of cells involved in the central nervous system damage, and that BP-reactivity is a component of the pathogenic response in MS.

In summary, the data presented demonstrate preferential Vβ gene usage among BP-specific clones derived from the blood of MS patients. The implication that those clones may be pathogenetically important suggests that the limited TCR repertoire provides a specific target for therapeutic intervention. Both monoclonal antibodies and synthetic TCR peptides specific for the V regions used could be considered in this approach.

**Table 7**

| TCR Vβ and Vα usage in Clones Derived from Three MS Patients | | | | | | | |
|---|---|---|---|---|---|---|---|
| BP-Specific Clones | | | | Non-BP Specific Clones | | | |
| Patient-Clone | Specificity Antigen (BP peptide) | Vα | Vβ | Patient-Clone | Antigen Specificity | Vα | Vβ |
| NL-6 | BP(NR) | NT' | 5.2 | MR-C1 | PPD | NT | 8.1 |
| NL-25 | BP(NR) | NT | 5.2>14 | MR-C2 | PPD | 15 | 6.1>14 |
| NL-27 | BP(NR) | NT | 5.2 | MR-C7 | PPD | NT | 13.2 |
| NL-41 | BP(NR) | NT | 5.2 | | | | |
| NL-46 | BP(45-89) | NT | 5.1 | WS-3B3 | nonspecific | 9 | 15 |
| NL-47 | BP(45-89) | 2,8 | 5.2 | WS-7D5 | nonspecific | 14 | 19 |
| NL-53 | BP(45-89) | 8 | 5.2 | WS-8D3 | nonspecific | 7 | 13.1 |
| NL-54 | BP(NR) | NT | 5.2 | WS-8C7 | nonspecific | 8 | 6.1>20 |
| NL-56 | BP(NR) | NT | 5.2 | WS-9D3 | nonspecific | 8>2 | 13.2>9 |
| NL-57 | BP(45-89) | NT | 5.2 | WS-13C7 | nonspecific | 1 | 13.1>10,6.1 |
| NL-59 | BP(45-89) | 8>6 | 5.2 | WS-6A2 | nonspecific | 8 | 10 |
| NL-63 | BP(NR) | NT | 5.2 | | | | |
| NL-97 | BP(1-38) | 2 | 5.2 | | | | |
| NL-101 | BP(NR) | NT | 5.2 | | | | |
| MR-21 | BP(NR) | NT | 5.2 | | | | |
| MR-22 | BP(90-170) | NT | 5.2 | | | | |
| MR-40 | BP(NR) | 7,11 | 6.1,9 | | | | |
| MR-41 | BP(90-170) | 1 | 5.2 | | | | |
| MR-43 | BP(45-89) | NT | 5.2 | | | | |
| MR-45 | BP(90-170) | 10 | 3 | | | | |
| MR-46 | BP(NR) | 1,14 | 9 | | | | |
| MR-48 | BP(90-170) | 7 | 19,15 | | | | |
| MR-51 | BP(NR) | 10 | 6.1 | | | | |
| MR-52 | BP(45-89) | 14 | 4 | | | | |
| WS-23 | BP(90-170) | 1 | 5.2 | | | | |
| WS-2B6 | BP(90-170) | 1 | 5.2 | | | | |
| WS-2C8 | BP(NR) | 7 | 12 | | | | |
| WS-2D5 | BP(NR) | 8 | 6.1>20,9 | | | | |
| WS-8D5 | BP(MR) | 10 | 6.1 | | | | |
| WS-14B7 | BP(NR) | NT | 5.2 | | | | |

### Table 7.

Clones were analyzed for Vβ and Vα expression by PCR and autoradiography as described in Figure 10. Human BP-reactive T cell lines were selected from the blood of MS patients as previously described (Chou et al., J. Neurosci. Res., in press (1991)). Briefly, peripheral blood mononuclear cells (PBMC) were cultured with 50 µg/ml human BP for 5 days prior to transfer into IL-2 rich medium. When growth in IL-2 showed, T cells were restimulated one time with 25 µg/ml Hu-BP presented by irradiated autologous PBMC, and cloned by limiting dilution two times as described (Chou et al., J. Neurosci. Res., in press (1991)). All sixteen clones from MS-patients and 15 of 15 clones from normal individuals analyzed by immunofluorescence expressed CD4. For some clones, more than one Vβ or Vα band could be identified, although usually one band predominated in intensity especially when a lower number (25-30) of PCR cycles was employed. The specificity of T cell clones for BP, BP peptides, or other antigens was assessed by proliferation as described Chou et al., J. Neurosci. Res., in press (1991)). Clones labeled as nonspecific were generated in the presence of BP but failed to demonstrate a proliferative response to BP above background (stimulator cells alone). All BP-reactive clones were tested for response to BP peptides 1-38, 45-89, and 90-170 prepared by Dr. Selene Chou as described (Chou et al., J. Neurochem.28:115-119 (1977)). NR indicates that a particular clone responded to BP but did not respond to any of these BP peptides. ^{†}NT - not tested.

**Table 8**

| Summary of Vβ Utilization in Clones from MS Patients and Controls | | | | | | |
|---|---|---|---|---|---|---|
| Patients | HLA type | Tested | Specificity | Vβ5.2 | vβ6.1 | Other Vβs (# clones positive) |
| NL | DR1,2:DQw1 | 14 | BP | 13 | 0 | Vβ5.1 |
| MR | DR2;DQw1 | 10 | BP | 4 | 2 | Vβ9(2x), 3, 4, 15, 19 |
| | | 3 | PPD | 0 | 1 | Vβ8.1, 13.2 |
| WS | DR2;DQw1 | 6 | BP | 3 | 2 | Vβ12 |
| | | 7 | nonspecific | 0 | 1 | Vβ131.1(2x), |
| | | | | | | 10, 13.2, 15, 19 |
| MD | DR2,4;DQw1,3 | 9 | BP | 4 | 1 | Vβ2, 7, 13.2, 15 |
| JH | DR7,-;DQ21.2,3 | 3 | BP | 2 | 4 | Vβ8,9 |
| | | | nonspecific | 0 | 0 | Vβ2 |
| | DR2,w6;DQw1 | 3 | BP | 0 | 1 | --* |
| | | 2 | nonspecific | 0 | 0 | Vβ4, 7 |
| | | 2 | HSV | 0 | 0 | Vβ4, 15 |
| RB | DR2,7:DQw1,2 | 1 | BP | 1 | 0 | none |
| **Total** | | **50** | **BP** | **27** | **10** | |
| | | **15** | **other** | **0** | **2** | |

| Controls | | | | | | |
|---|---|---|---|---|---|---|
| MA | DR3,w6;DQw1.2 | 13 | BP | 1 | 0 | Vβ14(11x),--* |
| | | 1 | nonspecific | 0 | 0 | Vβ13.1 |
| JT | DR7;DQw2 | 10 | BP | 0 | 3 | Vβ7(4x), 15, 18, 20 |
| BP | DR1.3;DQw1,2 | 7 | BP | 0 | 1 | Vβ7(4x),18,20 |
| LT | DR2.7;DQw1,2 | 6 | BP | 0 | 0 | Vβ14(2x), |
| | | | | | | 7, 12, 13.2, 17, 20 |
| DB | DR2:DQw1 | 4** | BP | 1 | 0 | --** |
| HY | DR4.7;DQw2,3 | 1 | BP | 0 | 0 | Vβ2 |
| | | 2 | nonspecific | 0 | 0 | Vβ13.1, 17 |
| **Total** | | **41** | **BP** | **2** | **4** | |
| | | **3** | **other** | **0** | **0** | |

### Table 8.

TCR Vα and Vβ expression was analyzed for T cell clones selected from the blood of seven MS patients and six normal individuals. Patients were being followed at the Oregon Health Sciences University MS clinic, and had clinically and laboratory supported definite MS. The patients had an average ambulation index of 3.2 ± 2.0 (range 2.6). Four patients had relapsing/remitting disease and three patients had chronic progressive disease. The normal individuals were from the Veterans Affairs Medical Center, selected on the basis of a positive proliferative response of PBMC to human BP in culture. All subjects were HLA-typed by standard serological methods at the Oregon Health Sciences University Transplantation laboratory. The uneven distribution of HLA DR2 expression (6 of 7 patients vs. 2 of 6 controls) reflects the frequency expected in MS patients versus the normal population.

In this analysis, only the predominant Vβ band for each clone is included. However, for some clones, 2 bands of nearly equal intensity are both recorded, and therefore the total number of Vβs may be greater than the number of clones analyzed. *No Vβ gene expression could be identified for those clones using the Vβ-specific oligomers described. ^{†}Three of these clones were only analyzed by monoclonal anti-Vβ antibodies (Kappler et al., Science 244:811-813 (1989)) directed to Vβ5.2/5.3, Vβ6.7, Vβ8.1, and Vβ12, and were negative.

### EXAMPLE III

### Sequence Homology Between V Gene Families In The CDR2 Region

In light of the predicted and observed high level of biological activity for the CDR2 region, the inventor decided to look further into the CDR2 region and line up the peptides so as to assay for homology.

A remarkable homology was witnessed at the NH2-terminus. Six out of eight residues were homologous with the two non-homologous ones at the C-terminus being conservative substitutions. This homology is noteworthy since, as discussed later, the clones selected against Vβ6.1 can recognize the Vβ5.2 epitope.

In an effort to further elucidate the relationship between the V gene families in the CDR2 region, the inventor also compared the Vβ14 epitope to Vβ12.2 and Vβ17.1. Ten out of the first twelve residues from the NH2-terminus are homologous. There is less homology at the C-terminus; four out of nine residues line up. The following Table indicates the sequence homology.

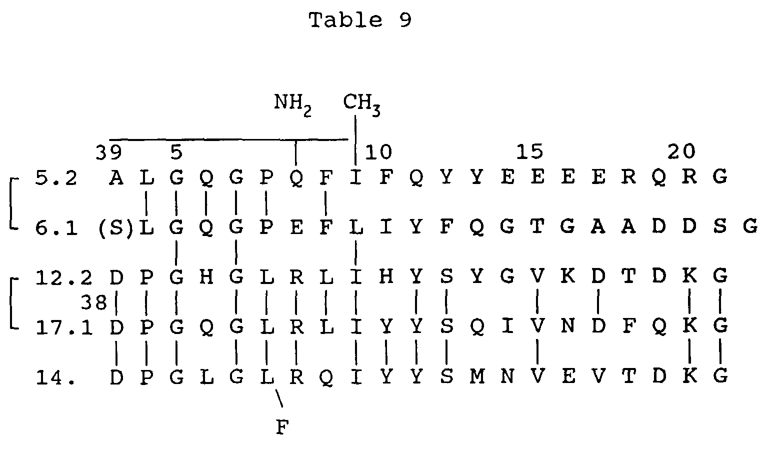

## Claims

1. A peptide consisting of an amino acid sequence corresponding to a portion of a T cell receptor, wherein the T cell receptor is a marker T cell receptor characteristic of a T cell mediated disease, the peptide being capable of stimulating regulatory T cells and inducing protection from the disease, wherein the peptide is selected from the group consisting of human Vβ5.2 (26-43), human Vβ5.2 (39-59) and human Vβ5.2 (59-78), human Vβ6.1 (1-22), human Vβ6.1 (39-59) and human Vβ6.1 (70-88) or a peptide substantially similar to a said peptide or a fragment thereof with the proviso that "the peptide substantially similar to a said peptide" and the "fragment thereof" are also capable of stimulating regulatory T cells and inducing protection from the disease.

2. A peptide consisting of an amino acid sequence corresponding to a portion of a T cell receptor, wherein the T cell receptor is a marker T cell receptor characteristic of a T cell mediated disease, the peptide being capable of stimulating regulatory T cells and inducing protection from the disease, wherein the amino acid sequence is selected from the group consisting of the consensus sequence ALGQGPXFX, the consensus sequence LRLIHYSYDVN and the consensus sequence DPGXGLRLIXYS or a peptide substantially similar to a said peptide or a fragment thereof with the proviso that "the peptide substantially similar to a said peptide" and the "fragment thereof" are also capable of stimulating regulatory T cells and inducing protection from the disease.

3. A peptide consisting of an amino acid sequence corresponding to a portion of a T cell receptor, wherein the T cell receptor is a marker T cell receptor characteristic of a T cell mediated disease, the peptide being capable of stimulating regulatory T cells and inducing protection from the disease, wherein the amino acid sequence is selected from the group consisting of the sequence ALGQGPQFIFQYYEEEERQRG, the sequence LGQGPEFLIYFQGTGAADDSG, the sequence DPGHGLRLIHYSYGVKDTDKG, the sequence DPGQGLRLIYYSQIVNDF QKG and the sequence DPGLGLFRQIYYSMNVEVTDKG or a peptide substantially similar to a said peptide or a fragment thereof with the proviso that "the peptide substantially similar to a said peptide" and the "fragment thereof" are also capable of stimulating regulatory T cells and inducing protection from the disease.

4. A peptide consisting of an idiotype which is cross-reactive with the peptide of claims 1, 2 or 3 or a peptide substantially similar to a said peptide or a fragment thereof with the proviso that "the peptide substantially similar to a said peptide" and the "fragment thereof" are also capable of stimulating regulatory T cells and inducing protection from the disease.

5. A peptide as claimed in any one of claims 1 to 4 conjugated to a carrier or antibody, which may be a monoclonal antibody.

6. A pharmaceutical composition comprising a peptide as claimed in any one of claims 1 to 5 in admixture with a pharmaceutically acceptable excipient.

7. A pharmaceutical composition as claimed in claim 6 comprising two or more of the peptides as claimed in any of claims 1 to 5, and wherein the two or more peptides are different.

8. A peptide as claimed in any one of claims 1 to 5, wherein the disease is multiple sclerosis.

9. The use of a peptide as defined in any one of claims 1 to 5 in the manufacture of a medicament for preventing, suppressing or treating a T cell mediated disease such as multiple sclerosis.

## Patentansprüche

1. Peptid bestehend aus einer Aminosäuresequenz, die einem Teil eines T-Zell-Rezeptors entspricht, wobei der T-Zell-Rezeptor ein Marker-T-Zell-Rezeptor ist, der für eine T-Zell-vermittelte Krankheit charakteristisch ist und das Peptid in der Lage ist, regulatorische T-Zellen zu stimulieren und einen Schutz gegen die Krankheit zu induzieren, und wobei das Peptid aus einer Gruppe ausgewählt ist, zu der ein menschliches Peptid Vβ5.2(26-43), ein menschliches Peptid Vβ5.2(39-59) und ein menschliches Peptid Vβ5.2(59-78), ein menschliches Peptid Vβ6.1(1-22), ein menschliches Peptid Vβ6.1(39-59) und menschliches Peptid Vβ6.1(70-88) oder ein Peptid, das dem besagten Peptid im Wesentlichen ähnlich ist, oder ein Fragment hiervon gehören, unter der Voraussetzung, dass das "Peptid das dem besagten Peptid im Wesentlichen ähnlich ist" und dass die "Fragmente hiervon" ebenfalls in der Lage sind, regulatorische T-Zellen zu stimulieren und einen Schutz gegen die Krankheit zu induzieren.

2. Peptid bestehend aus einer Aminosäuresequenz entsprechend einem Teil eines T-Zell-Rezeptor, wobei der T-Zell ein Marker T-Zell-Rezeptor ist, der für eine T-Zell-vermittelte Krankheit charakteristisch ist, das Peptid in der Lage ist regulatorische T-Zellen zu stimulieren und einen Schutz gegen die Krankheit zu induzieren, und wobei die Aminosäuresequenz aus einer Gruppe ausgewählt ist, zu der die Consensus-Sequenz ALGQGPXFX, die Consensus-Sequenz LRLIHYSYDVN und die Consensus-Sequenz DPGXGLRLIXYS oder ein Peptid, das dem besagten Peptid im Wesentlichen ähnlich ist, oder ein Fragment hiervon gehören, unter der Voraussetzung, dass das "Peptid das dem besagten Peptid im Wesentlichen ähnlich ist" und dass die "Fragmente hiervon" ebenfalls in der Lage sind, regulatorische T-Zellen zu stimulieren und einen Schutz gegen die Krankheit zu induzieren.

3. Peptid bestehend aus einer Aminosequenz entsprechend einem Teil eines T-Zell-Rezeptors, wobei der T-Zell-Rezeptor ein Marker T-Zell-Rezeptor ist, der für eine T-Zell-vermittelte Krankheit kennzeichnend ist, das Peptid in der Lage ist regulatorische T Zellen zu stimulieren und einen Schutz gegen die Krankheit zu induzieren, wobei die Aminosäuresequenz aus der Gruppe ausgewählt ist, zu der die Sequenz ALGQGPQFIFQYYEEEERQRG, die Sequenz LGQGPEFLIYFQGTGAADDSG, die Sequenz DPGHGLRLIHYSYGVKDTDKG, die Sequenz DPGQGLRLIYYSQIVNDFQKG und die Sequenz DPGLGLFRQIYYSMNVEVTDKG oder ein Peptid, das dem besagten Peptid im Wesentlichen ähnlich ist, oder ein Fragment hiervon gehören, unter der Voraussetzung, dass das "Peptid das dem besagten Peptid im Wesentlichen ähnlich ist" und dass die "Fragmente hiervon" ebenfalls in der Lage sind, regulatorische T-Zellen zu stimulieren und einen Schutz gegen die Krankheit zu induzieren.

4. Peptid bestehend aus einem Ideotyp, der mit dem Peptid nach den Ansprüchen 1, 2 oder 3 crossreaktiv ist, oder einem Peptid, das dem besagten Peptid im Wesentlichen ähnlich ist, oder einem Fragment hiervon, unter der Voraussetzung, dass das "Peptid das dem besagten Peptid im Wesentlichen ähnlich ist" und dass die "Fragmente hiervon" ebenfalls in der Lage sind, regulatorische T-Zellen zu stimulieren und einen Schutz gegen die Krankheit zu induzieren.

5. Peptid nach einem der Ansprüche 1 bis 4, das mit einem Träger oder einem Antikörper konjugiert ist, der ein monoklonaler Antikörper sein kann.

6. Pharmazeutische Zusammensetzung mit einem Peptid nach einem der Ansprüche 1 bis 5, gemischt mit einem pharmazeutisch verträglichen Excipienten.

7. Pharmazeutische Zusammensetzung nach Anspruch 6, mit zwei oder mehr Peptiden nach einem der Ansprüche 1 bis 5, wobei die beiden oder mehreren Peptide unterschiedlich sind.

8. Peptid nach einem der Ansprüche 1 bis 5, wobei die Krankheit Multiple Sklerose ist.

9. Verwendung eines Peptids, wie es in einem der Ansprüche 1 bis 5 definiert ist, zur Herstellung eines Medikamentes zur Prävention, Unterdrückung oder Behandlung einer T-Zell-vermittelten Krankheit wie Multipler Sklerose.

## Revendications

1. Peptide consistant en une séquence d'acides aminés correspondant à une partie d'un récepteur de lymphocytes T dans lequel le récepteur de lymphocytes T est un récepteur de lymphocytes T marqueur caractéristique d'une maladie médiée par les lymphocytes T, le peptide étant capable de stimuler des lymphocytes T de régulation et d'induire une protection contre la maladie, le peptide étant sélectionné parmi le groupe constitué par les peptides Vβ5.2 (26-43) humain, Vβ5.2 (39-59) humain et Vβ5.2 (59-78) humain, Vβ6.1 (1-22) humain, Vβ6.1 (39-59) humain et Vβ6.1 (70-88) humain ou un peptide pratiquement similaire audit peptide ou à un fragment de celui-ci sous réserve que 〈〈 le peptide pratiquement similaire audit peptide 〉〉 et les 〈〈 fragments de celui-ci 〉〉 soient aussi capables de stimuler les lymphocytes T de régulation et d'induire une protection contre ladite maladie.

2. Peptide consistant en une séquence d'acides aminés correspondant à une partie d'un récepteur de lymphocytes T dans lequel le récepteur de lymphocytes T est un récepteur de lymphocytes T marqueur caractéristique d'une maladie médiée par les lymphocytes T, le peptide étant capable de stimuler des lymphocytes T de régulation et d'induire une protection contre la maladie, la séquence d'acides aminés étant sélectionnée parmi le groupe constitué de la séquence consensuelle ALGQGPXFX, la séquence consensuelle LRLIHYSYDVN et la séquence consensuelle DPGXGLRLIXYS , ou un peptide pratiquement similaire audit peptide ou à un fragment de celui-ci sous réserve que 〈〈 le peptide pratiquement similaire audit peptide 〉〉 et les 〈〈 fragments de celui-ci 〉〉 soient aussi capables de stimuler les lymphocytes T de régulation et d'induire une protection contre ladite maladie.

3. Peptide consistant en une séquence d'acides aminés correspondant à une partie d'un récepteur de lymphocytes T dans lequel le récepteur de lymphocytes T est un récepteur de lymphocytes T marqueur caractéristique d'une maladie médiée par les lymphocytes T, le peptide étant capable de stimuler des lymphocytes T de régulation et d'induire une protection contre la maladie, la séquence d'acides aminés étant sélectionnée parmi le groupe constitué de la séquence ALGQGPQFIFQYYEEEERQRG , la séquence LGQGPEFLIYFQGTGAADDSG, la séquence DPGHGLRLIHYSYGVKDTDKG , la séquence DPGQGLRLIYYSQIVNDF Q K G et la séquence DPGLGLFRQIYYSMNVEVTDKG ou un peptide pratiquement similaire audit peptide ou à un fragment de celui-ci sous réserve que 〈〈 le peptide pratiquement similaire audit peptide 〉〉 et les 〈〈 fragments de celui-ci 〉〉 soient aussi capables de stimuler les lymphocytes T de régulation et d'induire une protection contre ladite maladie.

4. Peptide consistant en un idiotype présentant une réaction croisée avec le peptide de la revendication 1, 2 ou 3 ou peptide pratiquement similaire audit peptide ou à un fragment de celui-ci sous réserve que 〈〈 le peptide pratiquement similaire audit peptide 〉〉 et les 〈〈 fragments de celui-ci 〉〉 soient aussi capables de stimuler les lymphocytes T de régulation et d'induire une protection contre ladite maladie.

5. Peptide selon l'une des revendications 1 à 4 conjugué à un véhicule ou à un anticorps qui peut être un anticorps monoclonal.

6. Composition pharmaceutique comprenant un peptide selon l'une des revendications 1 à 5 sous forme d'un mélange avec un excipient pharmaceutiquement acceptable.

7. Composition pharmaceutique selon la revendication 6 comprenant deux ou davantage des peptides revendiqués à l'une quelconque des revendications 1 à 5 et dans laquelle les deux peptides, ou plus, sont différents.

8. Peptide selon l'une quelconque des revendications 1 à 5, la maladie étant la sclérose en plaque.

9. Utilisation d'un peptide tel que défini à l'une quelconque des revendications 1 à 5 dans la fabrication d'un médicament destiné à la prévention, la suppression ou le traitement d'une maladie médiée par les lymphocytes T telle que la sclérose en plaque.
